(19) Europäisches Patentamt
European Patent Office
Office européen des brevets

(11) Veröffentlichungsnummer: **0 372 385**
**A2**

(12) **EUROPÄISCHE PATENTANMELDUNG**

(21) Anmeldenummer: **89122074.1**

(22) Anmeldetag: **29.11.89**

(51) Int. Cl.5: **C07C 65/28, C07C 65/40, C07C 69/94, C07C 69/76**

(30) Priorität: **08.12.88 US 282100**

(43) Veröffentlichungstag der Anmeldung: **13.06.90 Patentblatt 90/24**

(84) Benannte Vertragsstaaten: **AT BE CH DE FR GB IT LI LU NL SE**

(71) Anmelder: **F. HOFFMANN-LA ROCHE AG**
**Postfach 3255**
**CH-4002 Basel(CH)**

(72) Erfinder: **Carson, Matthew**
**40 Brookfield Avenue**
**Nutley, N.J. 07110(US)**
Erfinder: **Han, Ru-Jen Lee**
**17 Colonia Avenue**
**Princeton Junction, N.J. 08550(US)**
Erfinder: **LeMahieu, Ronald Andrew**
**18 Spruce Road**
**North Calwell, N.J. 07006(US)**

(74) Vertreter: **Grossner, Lutz, Dr. et al**
**Grenzacher Strasse 124 Postfach 3255**
**CH-4002 Basel(CH)**

(54) **Neue Naphthalincarbonsäuren, deren Herstellung und Verwendung als Heilmittel.**

(57) Naphthalincarbonsäurederivate der allgemeinen Formel

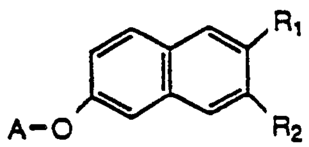

I

worin einer der Reste $R_1$ und $R_2$ $-(CHR_{10})_m-C(O)OR$ und der andere Wasserstoff ist, R Wasserstoff oder Niederalkyl, $R_{10}$ Wasserstoff oder Niederalkyl und m 0 oder 1 ist, A

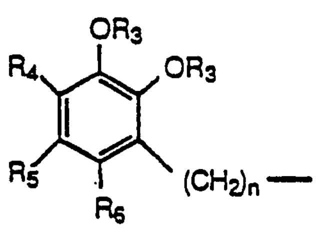

A'

ist, worin $R_3$ Wasserstoff oder Acyl, $R_4$ Wasserstoff, Halogen, Niederalkyl, Aryl oder Cycloalkyl, $R_5$ und $R_6$ unabhängig voneinander Wasserstoff oder Halogen und n eine ganze Zahl von 2-10 ist oder A ein Rest

EP 0 372 385 A2

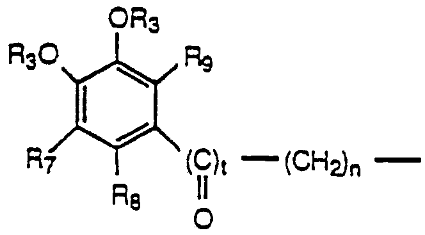

A''

ist, worin $R_3$ Wasserstoff oder Acyl, $R_7$ Wasserstoff oder Niederalkyl, $R_8$ und R9 unabhängig voneinander Wasserstoff, Niederalkyl oder Halogen, t O oder 1 und n eine ganze Zahl von 2-10 ist
und falls $R_{10}$ Niederalkyl ist, Enantiomere und Racemate davon und, wenn R Wasserstoff ist, Salze davon mit pharmazeutisch anwendbaren Basen, können als Heilmittel, insbesondere zur Behandlung entzündlicher Erkrankungen, z.B. Arthritis oder Psoriasis Verwendung finden.

## Neue Naphthalincarbonsäuren, deren Herstellung und Verwendung als Heilmittel

Die vorliegende Erfindung betrifft substituierte Naphthalincarbonsäurederivate der Formel

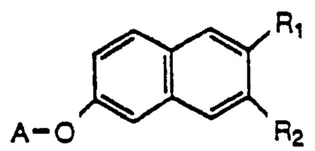

I

worin einer der Reste $R_1$ und $R_2$ $-(CHR_{10})_m-C(O)OR$ und der andere Wasserstoff ist, R Wasserstoff oder Niederalkyl, $R_{10}$ Wasserstoff oder Niederalkyl und m 0 oder 1 ist, A

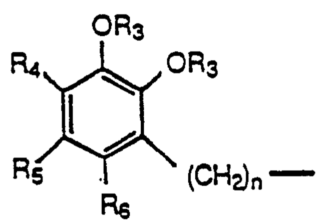

A'

ist, worin $R_3$ Wasserstoff oder Acyl, $R_4$ Wasserstoff, Halogen, Niederalkyl, Aryl oder Cycloalkyl, $R_5$ und $R_6$ unabhängig voneinander Wasserstoff oder Halogen und n eine ganze Zahl von 2-10 ist oder A ein Rest

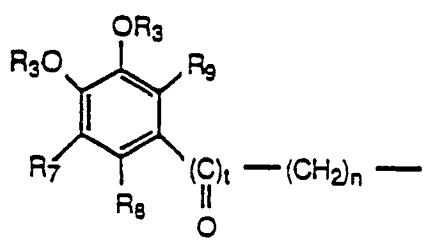

A"

ist, worin $R_3$ Wasserstoff oder Acyl, $R_7$ Wasserstoff oder Niederalkyl, $R_8$ und $R_9$ unabhängig voneinander Wasserstoff, Niederalkyl oder Halogen, t O oder 1 und n eine ganze Zahl von 2-10 ist und falls $R_{10}$ Niederalkyl ist, Enantiomere und Racemate davon und, wenn R Wasserstoff ist, Salze davon mit pharmazeutisch anwendbaren Basen.

Die Verbindungen der Formel I und, wenn R Wasserstoff ist, Salze davon mit pharmazeutisch anwendbaren Basen sind als Mittel zur Behandlung entzündlicher Erkrankungen wie Arthritis, entzündlicher Darmerkrankungen, wie Colitis, Hautkrankungen wie Psoriasis (bei topischer Verabreichung) und bronchopulmonärer Erkrankungen, wie Asthma von Wert.

Der hier verwendete Ausdruck "Niederalkyl" bezeichnet einen geradkettig oder verzweigten gesättigten Kohlenwasserstoffrest mit 1-7 C-Atomen, beispielsweise Methyl, Aethyl, Propyl, Isopropyl, Butyl, t-Butyl, Neopentyl, Pentyl, Heptyl. Verzweigte gesättigte Kohlenwasserstoffe sind für die Reste $R_4$ und $R_7$ bevorzugt. Der Ausdruck "Halogen" bezeichnet alle Halogene, d.h. Brom, Chlor, Fluor und Jod. Der Ausdruck "Aryl" bezeichnet Phenyl oder Phenyl, das einen oder zwei Substituenten enthält, die unabhängig voneinander aus der Gruppe Halogen, Trifluormethyl, Niederalkyl, Niederalkoxy, Nitro, Amino, Niederalkylamino und di-Niederalkylamino ausgewählt sein können. Der Ausdruck "Acyl" bezeichnet eine Alkanoylgruppe, die sich von einer aliphatischen Carbonsäure mit 1-7 C-Atomen ableitet, beispielsweise Formyl, Acetyl und Propionyl und Aroylgruppen, die sich von aromatischen Carbonsäuren ableiten wie Benzoyl. Der Ausdruck "Cycloalkyl" bezeichnet vorzugsweise einen cyclischen Kohlenwasserstoff mit 3-6 C-Atomen, der unsubstituiert oder durch Niederalkyl substituiert sein kann und besonders bevorzugt 5-6 C-Atome enthält, wie Cyclopropyl, Cyclopentyl und Cyclohexyl.

Die Verbindungen der Formel I lassen sich auch durch die Formel Ia und Ib darstellen, je nachdem ob

der Rest A eine Gruppe A′ oder A″ bedeutet

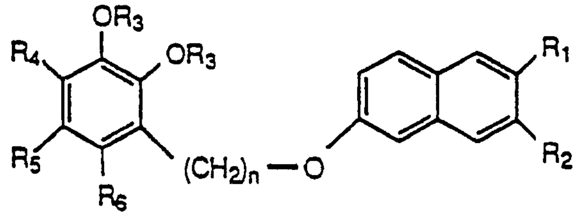

Ia

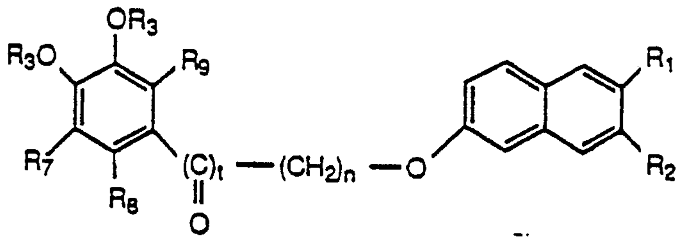

Ib

wobei $R_1$, $R_2$, $R_3$, $R_4$, $R_5$, $R_6$, $R_7$, $R_8$, $R_9$, t und n die obige Bedeutung haben.

Bevorzugte Verbindungen der Formel Ia sind diejenigen in denen A A′ ist, $R_1$ $-(CHR_{10})_m-C(O)OR$, $R_2$ Wasserstoff, $R_{10}$ Wasserstoff oder Methyl, $R_3$ Wasserstoff und R, $R_4$, $R_5$, $R_6$, m and n die obige Bedeutung haben. Insbesondere solche, in denen $R_1$ $-(CHR_{10})_mC(O)OR$, $R_{10}$ Wasserstoff oder Methyl, $R_3$ Wasserstoff ist und R, $R_7$, $R_8$, $R_9$, m, t und n die obige Bedeutung haben.

Die am meisten bevorzugten erfindungsgemässen Verbindungen sind:
(S)-6-[6-(2,3-Dihydroxyphenyl)hexyloxy]-α-methyl-2-naphthalinessigsäure;
(S)-6-[6-(2,3-Dihydroxyphenyl)hexyloxy]-α-methyl-2-naphthalinessigsäureäthylester;
(Rac)-6-[6-(2,3-Dihydroxyphenyl)hexyloxy]-α-methyl-2-naphthalinessigsäure;
6-[6-(2,3-Dihydroxyphenyl)hexyloxy]-2-naphthalinessigsäure;
6-[6-(2,3-Dihydroxyphenyl)hexyloxy]-2-naphthalincarbonsäure und
(S)-6-[6-(3,4-Dihydroxyphenyl)hexyloxy]-α-methyl-2- naphthalinessigsäure.

Beispiele weiterer erfindungsgemässer Verbindungen sind:
(S)-6-[4-(2,3-Dihydroxyphenyl)butoxy]-α-methyl-2-naphthalinessigsäure;
6-[4-(2,3-Dihydroxyphenyl)butoxy]-2-naphthalincarbonsäure;
(R)-6-[6-(2,3-Dihydroxyphenyl)hexyloxy]-α-methyl-2-naphthalinessigsäure;
(rac)-6-[4-(2,3-Dihydroxyphenyl)butoxy]-α-methyl-2-naphthalinessigsäure;
6-[4-(2,3-Dihydroxyphenyl)butoxy]-2-naphthalincarbonsäureäthylester
(S)-7-[6-(2,3-Dihydroxyphenyl)hexyloxy]-α-methyl-2-naphthalinessigsäure;
7-[6-(2,3-Dihydroxyphenyl)hexyloxy]-2-naphthalincarbonsäure;
(R)-7-[6-(2,3-Dihydroxyphenyl)hexyloxy]-α-methyl-2-naphthalinessigsäure;
(S)-6-[8-(2,3-Dihydroxyphenyl)octyloxy]-α-methyl-2-naphthalinessigsäure;
(S)-6-[6-(2,3-Diacetoxyphenyl)hexyloxy]-α-methyl-2-naphthalinessigsäure;
(S)-6-[6-[2,3-Dihydroxy-4-(1-methylethyl)phenyl]hexyloxy]-α-methyl-2-naphthalinessigsäure;
(S)-6-[4-[2,3-Dihydroxy-4-(1,1-dimethylethyl)phenyl]butoxy]-α-methyl-2-naphthalinessigsäure;
(R)-6-[6-[2,3-Dihydroxy-4-(1-methylethyl)phenyl]hexyloxy]-α-methyl-2-naphthalinessigsäure;
7-[4-(2,3-Dihydroxyphenyl)butoxy]-2-naphthalincarbonsäure;
(S)-6-[5-(6-Chloro-2,3-dihydroxyphenyl)pentyloxy]-α-methyl-2-naphthalinessigsäure;
(S)-6-[5-(5,6-Dichloro-2,3-dihydroxyphenyl)pentyloxy]-α-methyl-2-naphthalinessigsäure;
(S)-6-[5-(2,3-Dihydroxy-4,5,6-trichlorophenyl)pentyloxy]-α-methyl-2-naphthalinessigsäure;
(S)-7-[5-(2,3-Dihydroxy-4,5,6-trichlorophenyl)pentyloxy]- α-methyl-2-naphthalinessigsäure;
6-[5-(2,3-Dihydroxy-4,5,6-trichlorophenyl)pentyloxy]-2-naphthalincarbonsäure;
(R)-6-[5-(2,3-Dihydroxy-4,5,6-trichlorophenyl)pentyloxy]-α-methyl-2-naphthalinessigsäure;
(S)-6-[8-(3,4-Dihydroxyphenyl)octyloxy]-α-methyl-2-naphthalinessigsäure;
(S)-6-[4-(3,4-Dihydroxyphenyl)butoxy-α-methyl-2-naphthalinessigsäure;
6-[4-(3,4-Dihydroxyphenyl)butoxy]-2-naphthalincarbonsäure;
(R)-6-[6-(3,4-Dihydroxyphenyl)hexyloxy]-α-methyl-2-naphthalinessigsäure;

7-[4-(3,4-Dihydroxyphenyl)butoxy]-2-naphthalincarbonsäure;
(S)-6-[[6-(3,4-Dihydroxyphenyl)-6-oxohexyl]oxy]-α-methyl-2-naphthalinessigsäure;
6-[[4-(3,4-Dihydroxyphenyl-4-oxobutyl]oxy]-2-naphthalinessigsäureäthylester;
(S)-6-[6-(3,4-Dihydroxy-2,5-dimethylphenyl)hexyloxy]-α-methyl-2-naphthalinessigsäure;
7-[6-(6-(3,4-Dihydroxy-2,5-dimethylphenyl)hexyloxy]-2-naphthalincarbonsäure;
6-[6-(3,4-Dihydroxy-2,5-dimethylphenyl)hexyloxy]-2-naphthalincarbonsäure;
6-[[6-(3,4-Dihydroxy-2,5-dimethylphenyl)-6-oxohexyl]oxy]-2-naphthalinessigsäure:
6-[4-(2,3-Dihydroxyphenyl)butoxy]-2-naphthalinessigsäure
6-[6-[3,4-Dihydroxy-5-(1-methylethyl)phenyl]hexyloxy]-2-naphthalinessigsäure;
6-[6-(3,4-Dihydroxy-6-fluorophenyl)hexyloxy]-2-naphthalinessigsäure;
6-[6-(2,3-Dihydroxyphenyl)hexyloxy]-2-naphthalinessigsäureäthylester;

Die Verbindungen der Formel I und deren Salze können erfindungsgemäss dadurch hergestellt werden, dass man

(a) eine Verbindung der Formel

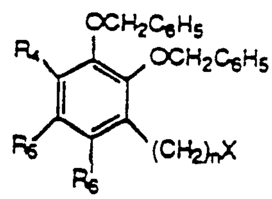

V

mit einer Verbindung der Formel

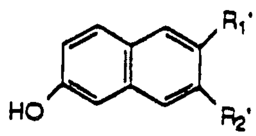

XXIII

zu einer Verbindung der Formel

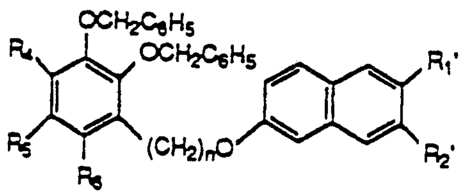

XXIVa

oder

(b) eine Verbindung der Formel

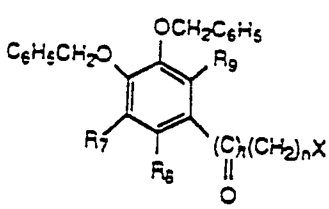

XXVI

mit einer Verbindung der Formel

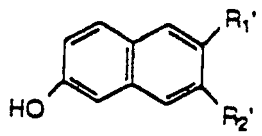

XXIII

zu einer Verbindung der Formel

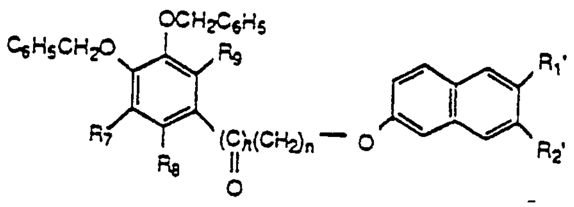

XXVII

umsetzt, wobei in den obigen Formeln einer der Reste $R_1'$ und $R_2'$ Wasserstoff und der andere $-(CHR_{10})_mCOOR'$ darstellt, worin $R'$ Niederalkyl ist, X Halogen ist und $R_4$, $R_5$ und $R_6$ m, n und t die obige Bedeutung haben; worauf man in beliebiger Reihenfolge die Benzyläthergruppen der Verbindungen der Formel XXIVa und XXVII und, gewünschtenfalls, eine Alkylestergruppe in den Gruppen $R_1'$ und $R_2'$ entfernt um eine Verbindung der Formel I zu erhalten worin $R_3$ Wasserstoff ist und $R_1$, $R_2$, $R_4$, $R_5$, $R_6$, m, n und t die obige Bedeutung haben und gewünschtenfalls phenolische Hydroxygruppen in der erhaltenen Verbindung der Formel I acyliert und weiterhin gewünschtenfalls eine erhaltene Carbonsäure in ein Salz überführt.

Die Herstellung der Verbindungen der Formel I und Zwischenprodukte dafür ist ausführlicher in den nachstehenden Reaktionsschemata I-VII beschrieben.

REAKTIONSSCHEMA I

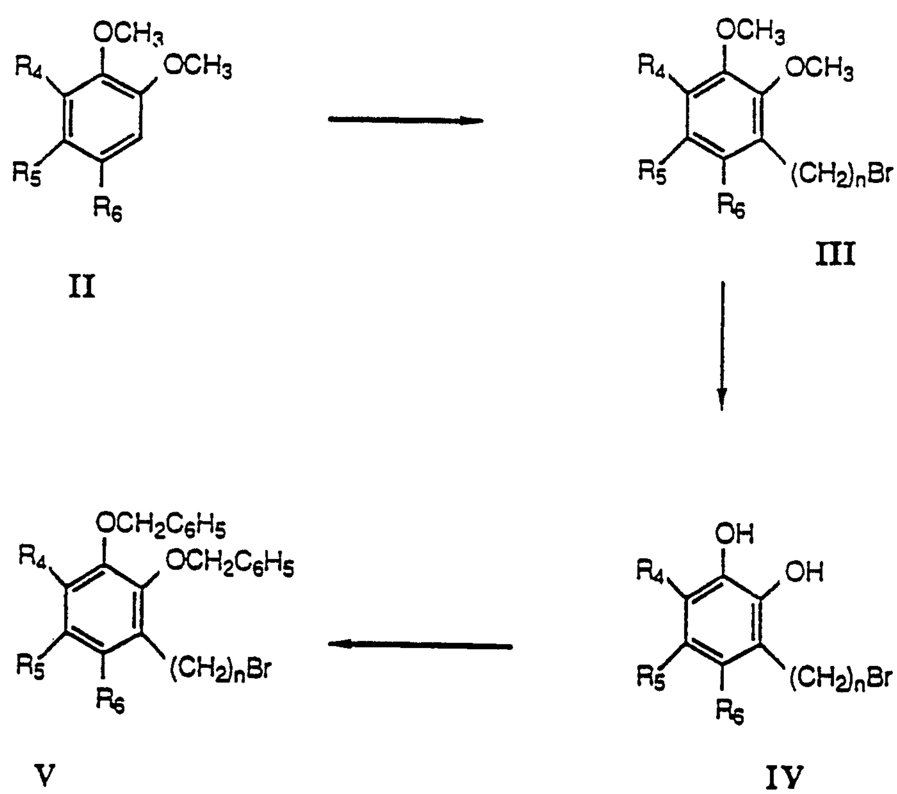

II

III

IV

V

worin $R_4$, $R_5$, $R_6$ und n die obige Bedeutung haben.

Im Reaktionsschema I wird eine Verbindung der Formel II, die zu bekannten Verbindung gehört oder nach an sich bekannten Verfahren hergestellt werden kann, in die entsprechenden bekannten Verbindungen der Formel II übergeführt wie in J. Chem. Soc. Perkin I, 2331 (1980) beschrieben. Insbesondere wird eine Verbindung der Formel II mit einem Lithiumalkyl, vorzugsweise Butyllithium, in Gegenwart eines Lösungsmittels wie Diäthyläther oder Tetrahydrofuran bei Temperaturen im Bereich von etwa -75° bis 0° C in das entsprechende Lithiumsalz übergeführt, das in situ mit überschüssigem Dibromalkan bei Temperaturen im Bereich von etwa 0° bis 50° C umgesetzt wird.

Eine Verbindung der Formel III kann in die entsprechende Verbindung der Formel IV, beispielsweise durch Behandlung mit Bortribromid in einen halogenierten Kohlenwasserstoff, beispielsweise Chloroform oder 1,2-Dichloräthan oder vorzugsweise Methylenchlorid bei Temperaturen im Bereich von etwa -75° bis etwa 25° C übergeführt werden.

Die erhaltene Verbindung der Formel IV kann in die entsprechende Verbindung der Formel V in Gegenwart von Benzylchlorid oder Benzylbromid, Kaliumjodid oder Natriumjodid und einem Alkalimetallcarbonat, beispielsweise Natrium- oder Kaliumcarbonat, in einem Lösungsmittel wie Aceton oder Methyläthylketon bei Rückflusstemperatur oder mit Dimethylformamid bei Temperaturen im Bereich von etwa 50° bis etwa 100° C umgewandelt werden.

REAKTIONSSCHEMA II

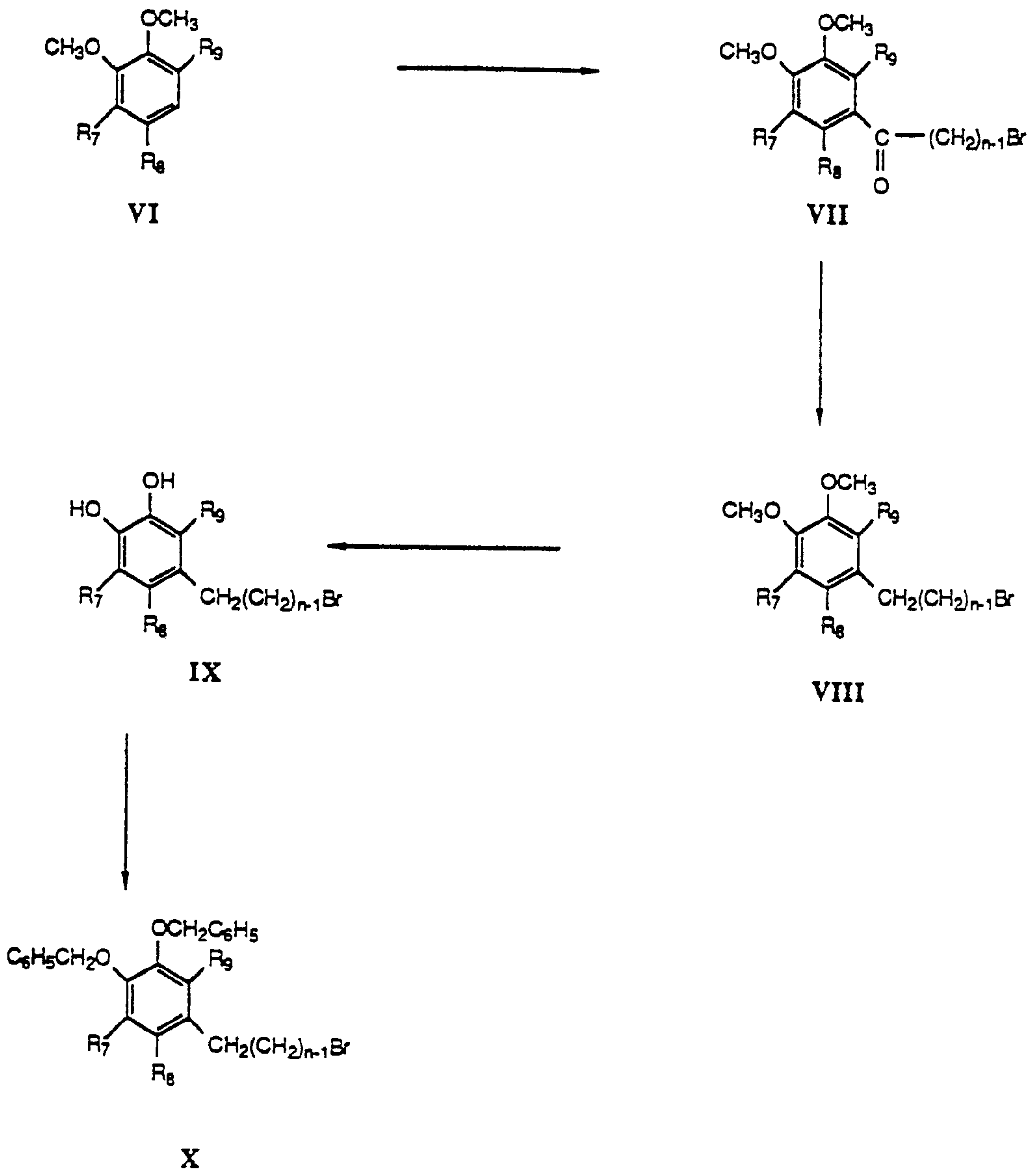

VI

VII

VIII

IX

X

worin $R_7$, $R_8$, $R_9$ und n die obige Bedeutung haben.

Im Reaktionsschema II wird eine Verbindung der Formel VI die zu bekannten Verbindungen gehört oder nach an sich bekannten Verfahren hergestellt werden kann, in eine Verbindung der Formel VII mit üblichen Acylierungsverfahren umgewandelt werden, beispielsweise durch Behandlung mit einer Bromsäure und Trifluoracetanhydrid bei einer Temperatur im Bereich von 25° bis etwa 40° C ohne Anwendung eines Lösungsmittels oder in Gegenwart eines Lösungsmittels wie Methylenchlorid oder 1,2-Dichloräthan.

Alternativ kann ein Bromsäurechlorid und Aluminiumchlorid in einem Lösungsmittel wie Methylenchlorid oder 1,2-Dichloräthan bei einer Temperatur im Bereich von etwa 0° bis etwa 40° C angewandt werden.

Die Reduktion einer Verbindung der Formel VII zur entsprechenden Verbindung der Formel VIII kann durch Hydrierung in einem Parr-Hydrierapparat bei Wasserstoffdrucken von etwa 3,4 bis etwa 4 bar mittels eines Palladiumkatalysators in einem Lösungsmittel wie Aethanol, Aethylacetat oder Tetrahydrofuran bei Temperaturen im Bereich von etwa 25° bis etwa 70° C durchgeführt werden. Zusätzlich zu dem Palladiumkatalysator kann ein Mineralsäurekatalysator zugesetzt werden.

Die Umwandlung einer Verbindung der Formel VIII in eine Verbindung der Formel IX und dann in eine Verbinung der Formel X kann in analoger Weise ausgeführt werden wie im Reaktionsschema I für die Umwandlung von III in IV und V beschrieben.

REAKTIONSSCHEMA III

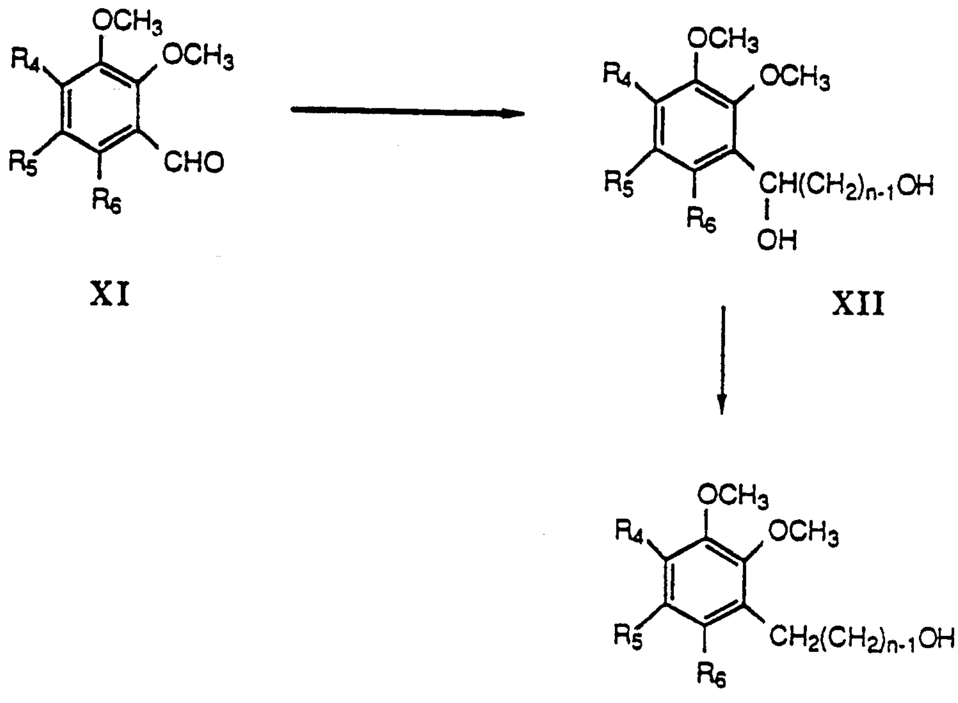

XI

XII

XIII

worin $R_4$, $R_5$, $R_6$ und n die obige Bedeutung haben.

Im Reaktionsschema III kann ein Aldehyd der Formel XI, der zu bekannten Verbindungen gehört oder nach an sich bekannten Verfahren hergestellt werden kann in die entsprechende Verbindung der Formel XII umgewandelt werden gemäss Chem. Ind., 526 (1974). Insbesondere kann man den Aldehyd der Formel XII mit einem Lithiumreagens nach Standardverfahren in einem Lösungsmittel wie Aethyläther oder Tetrahydrofuran bei Temperaturen im Bereich von etwa -20° bis etwa 35° C umsetzen. Die Alkoholschutzgruppe kann aus dem Produkt durch Behandlung mit verdünnter Salzsäure bei etwa 25° abgespalten werden, wobei das Diol der Formel XII erhalten wird.

Anschliessende Hydrogenolyse einer Verbindung der Formel XII durch Schütteln in einem Parr-Hydrierapparat unter Wasserstoffdrucken von etwa 2,7 bis 4 bar mittels eines Palladiumkatalysators bei Temperaturen im Bereich von etwa 25 bis etwa 50° C in einem Lösungsmittel, wie Aethylacetat, Aethanol oder Tetrahydrofuran gibt die entsprechende Verbindung der Formel XIII.

Eine Verbindung der Formel XIII kann in eine Verbindung der Formel V umgewandelt werden, in ähnlicher Weise wie im Reaktionsschema I bei der Umwandlung der Verbindung III in IV und V, d.h. durch Behandlung mit Bortribromid und anschliessende Benzylierung.

REAKTIONSSCHEMA IV

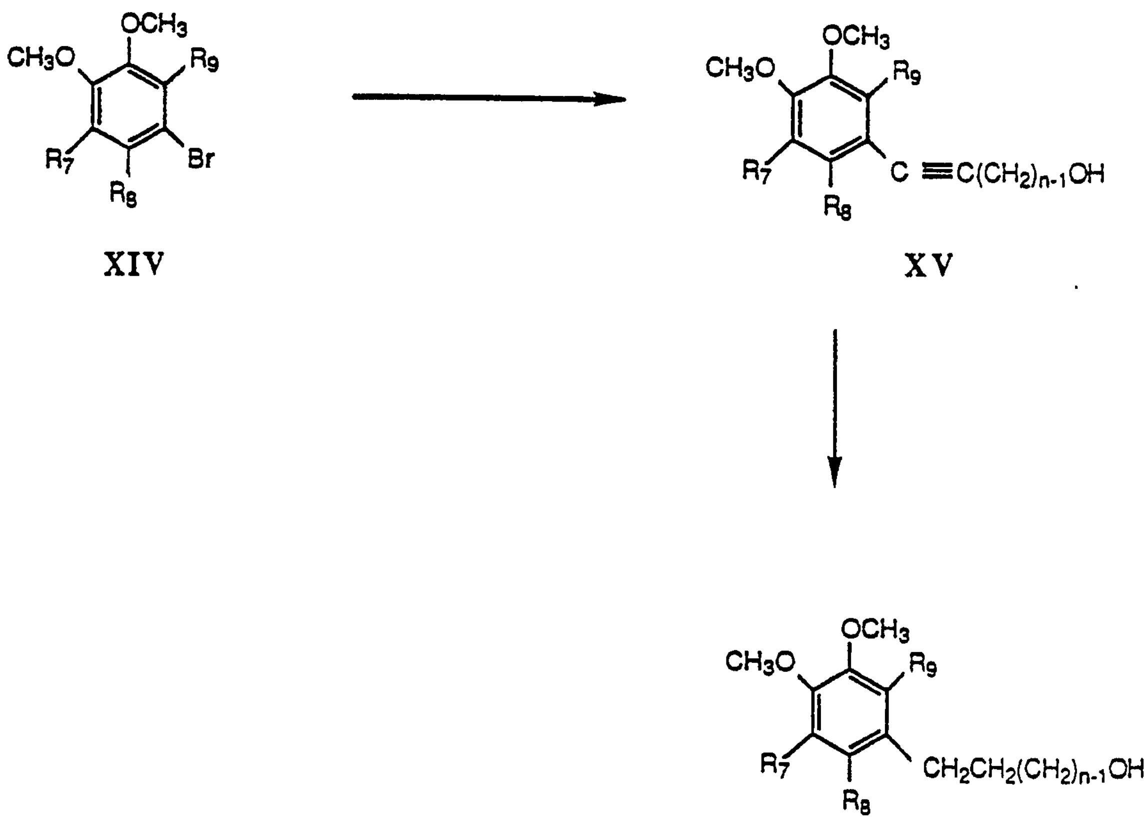

XIV

XV

XVI

worin $R_7$, $R_8$, $R_9$ und n die obige Bedeutung haben.

Im Reaktionsschema IV wird eine Verbindung der Formel XIV in den Acetylenalkohol der Formel XV durch Umsatz mit einem Acetylenalkohol in Gegenwart eines bis-(Triphenylphosphin) palladiumdichlorids, Kupfer-(I)-jodid und einem organischen Amin (Triäthylamin) wie in Tet. Letters, 4467 (1975) umgewandelt.

Die Reaktion wird in einem Lösungsmittel, beispielsweise einem halogenierten Kohlenwasserstoff wie Methylenchlorid, Chloroform oder 1,2-Dichloräthan bei Temperaturen im Bereich von etwa 25° bis etwa 50° C durchgeführt.

Die erhaltene Verbindung der Formel XV wird in eine Verbindung der Formel XVI nach Standardverfahren, beispielsweise katalytische Hydrierung bei Atmosphärendruck und Raumtemperatur, umgewandelt.

Eine Verbindung der Formel XVI kann in eine Verbindung der Formel X in ähnlicher Weise umgewandelt werden wie im Reaktionsschema I bei der Umwandlung der Verbindung III in IV und V, d.h. durch Behandlung mit Bortribromid und anschliessende Benzylierung.

REAKTIONSSCHEMA V

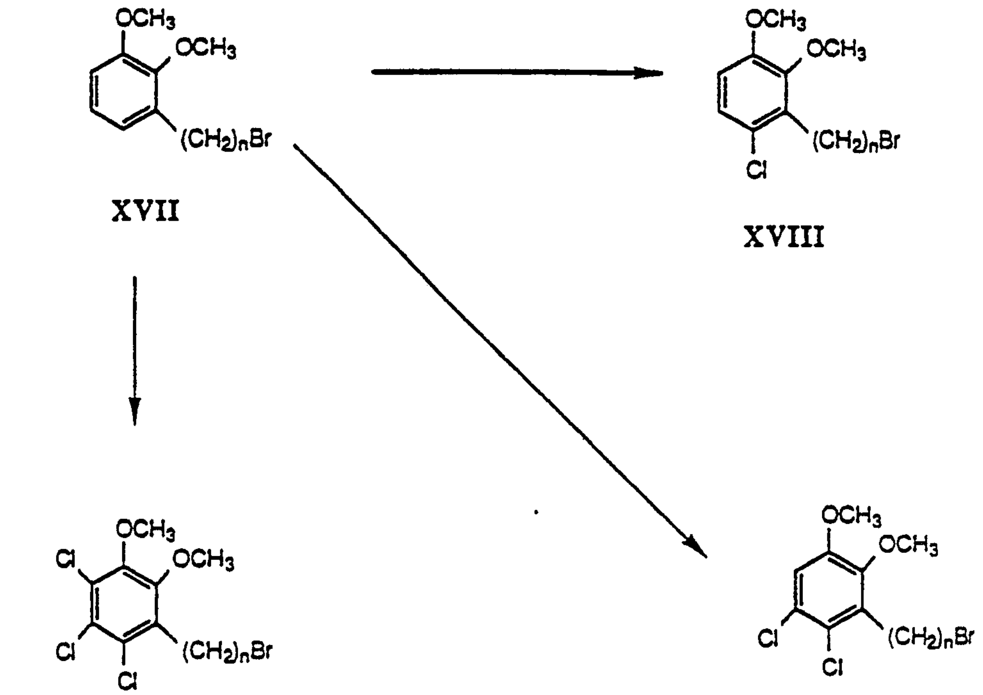

XVII

XVIII

XX

XIX

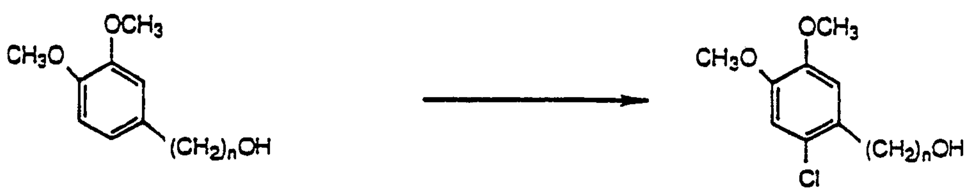

XXI

XXII

worin n die obige Bedeutung hat.

Im Reaktionsschema V wird eine Verbindung der Formel XVII, die zu bekannten Verbindungen gehört oder nach an sich bekannten Verfahren hergestellt werden kann, in die entsprechende Monochlorverbindung der Formel XXVIII, die Dichlorverbindung der Formel XXIX und die Trichlorverbindung der Formel XX durch Behandlung mit einer entsprechenden Menge Chlor in einem inerten Lösungsmittel, wie einem chlorierten Kohlenwasserstoff, beispielsweise Methylenchlorid, Chloroform oder 1,2-Dichlormethan, bei Temperaturen im Bereich von etwa -20° bis etwa 25° C übergeführt.

Die Umwandlung einer Verbindung der Formel XXI die zu bekannten Verbindungen gehört oder nach an sich bekannten Verfahren hergestellt werden kann in eine entsprechende Verbindung der Formel XXII, kann unter den oben beschriebenen Reaktionsbedingungen ausgeführt werden.

REAKTIONSSCHEMA VI

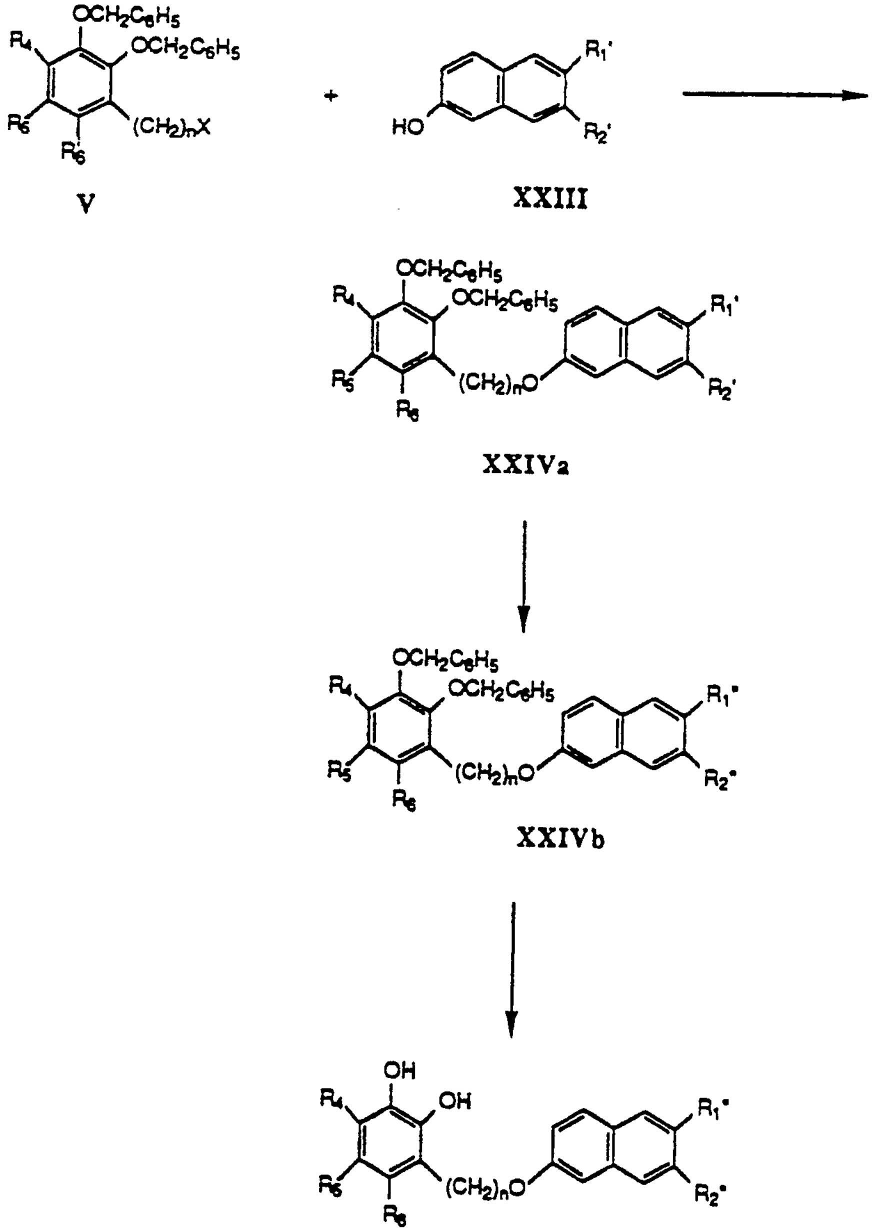

V XXIII

XXIVa

XXIVb

Ia'

worin X Halogen ist, einer der Reste $R_1'$ und $R_2'$ Wasserstoff und der andere $-(CHR_{10})_m-COOR'$ ist, worin $R'$ Niederalkyl ist, wobei die (R), (S) und (RS)-Konfiguration inbegriffen ist, wenn $R_{10}$ Niederalkyl ist, einer der Reste $R_1''$ und $R_2''$ Wasserstoff und der andere $-(CHR_{10})_m-COOR''$ ist, worin $R''$ Wasserstoff oder Niederalkyl ist, wobei die (R), (S) und (RS)-Konfiguration inbegriffen ist, wenn $R_{10}$ Niederalkyl ist und wobei $R_4$, $R_5$, $R_6$ und $R_{10}$ die obige Bedeutung haben.

Im Reaktionsschema VI wird eine Verbindung der Formel V mit einer Verbindung der Formel XXIII umgesetzt, die zu bekannten Verbindungen gehört oder nach an sich bekannten Verfahren hergestellt werden kann, wobei die entsprechende Verbindung der Formel XXIVa erhalten wird. Die Reaktion wird in Gegenwart eines Alkalimetallcarbonats als Base, beispielsweise Natriumcarbonat, vorzugsweise Kaliumcarbonat, unter Zusatz von Natriumjodid oder Kaliumjodid in einem Lösungsmittel wie Aceton, Methyläthylketon, Dimethylformamid oder Toluol, bei Temperaturen im Bereich von etwa 40° bis etwa 70° C durchgeführt. Der Fest-Flüssigphasen-Transferkatalysator tris[2-(2-Methoxyäthoxy)äthyl]amin kann angewandt werden um die Reaktion zu erleichtern, wenn Toluol als Lösungsmittel verwendet wird.

Hydrolyse der Verbinung der Formel XXIVa zur entsprechenden Verbindung der Formel XXIVb kann nach Standardverfahren durchgeführt werden, beispielsweise mittels Alkalimetallhydroxiden, wie Natriumhydroxid oder Kaliumhydroxid in Lösungsmitteln wie Methanol oder Aethanol, manchmal unter Zusatz von Dioxan als Lösungshilfe bei Temperaturen im Bereich von etwa 25° bis etwa 65° C.

Die Umwandlung einer Verbindung der Formel XXXIVb in die entsprechende Verbindung der Formel Ia′ kann nach Standardverfahren, beispielsweise durch katalytische Hydrierung bei Atmosphärendruck und Raumtemperatur ausgeführt werden. Die erhaltene Verbindung der Formel Ia wird abgetrennt und nach konventionellen Verfahren gereinigt, beispielsweise durch Ausfällen, Kristallisieren oder Chromatographie.

REAKTIONSSCHEMA VII

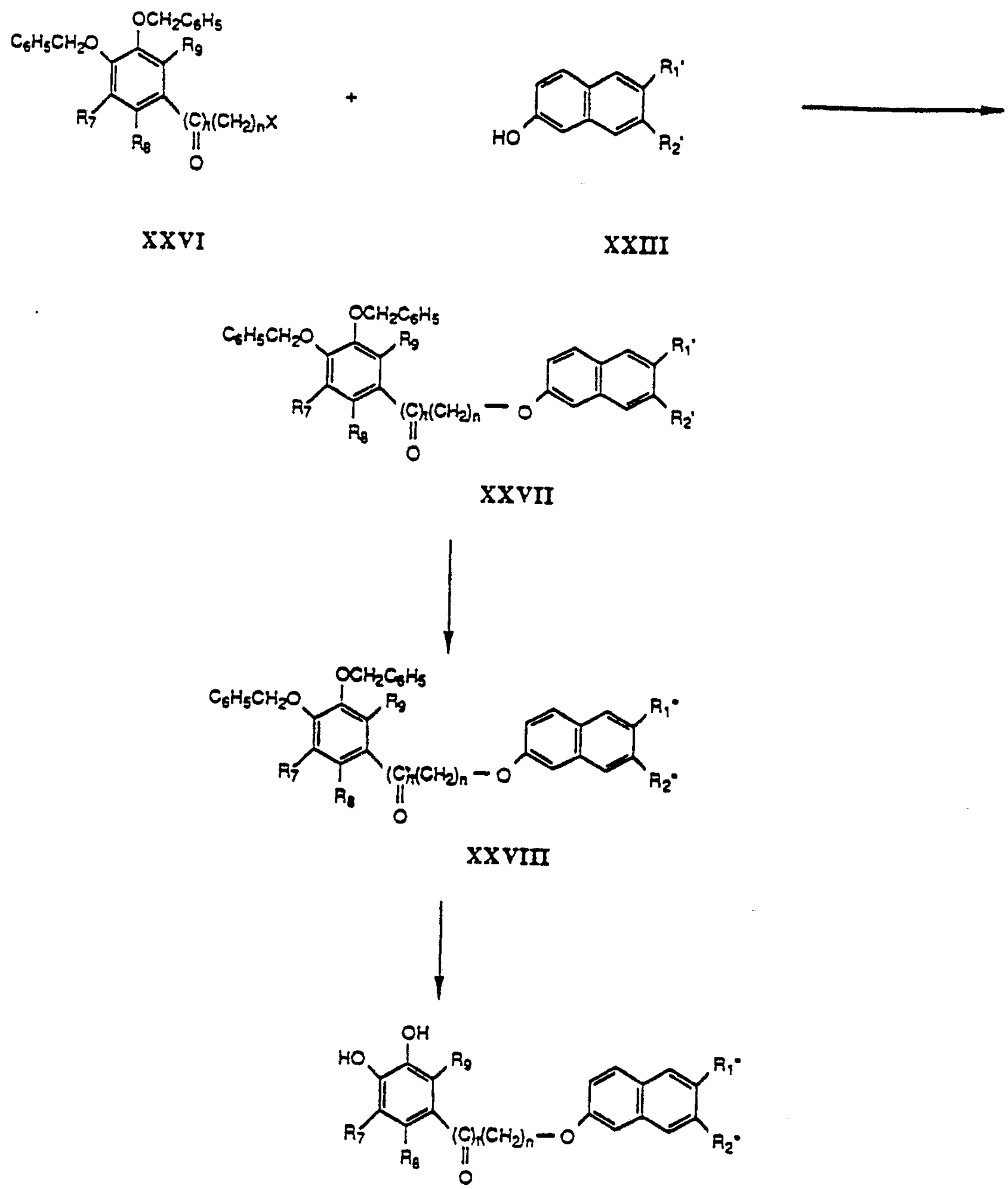

XXVI XXIII XXVII XXVIII Ib′

worin X, $R_1'$, $R_2'$, $R_1''$, $R_2''$, $R_7$, $R_8$, $R_9$, n und $t_1$ die obige Bedeutung haben.

Im Reaktionsschema VII wird eine Verbindung der Formel XXVI mit einer Verbindung der Formel XXVIII zu einer Verbindung der Formel XXVII umgesetzt. Die Reaktion wird unter den Bedingungen durchgeführt, die im Reaktionsschema VI für die Umsetzung einer Verbindung der Formel XXIII zu einer Verbindung der

Formel XXIVa beschrieben sind. Die Hydrolyse einer Verbindung der Formel XXVII zu einer Verbindung der Formel XXVIII kann ebenfalls wie im Reaktionsschema VI für die Umwandlung der Verbindung der Formel XXIVa in die Verbindung XXIVb beschrieben ausgeführt werden.

Die Umwandlung einer Verbindung der Formel XXVIII in die entsprechende Verbindung Ib′ kann durch Hydrierung bei Wasserstoffdrucken von etwa 3,4 bis 4 bar mittels Palladiumkatalysatoren in einem Lösungsmittel wie Aethanol, Aethylacetat oder Tetrahydrofuran bei Temperaturen im Bereich von etwa 25° bis etwa 70° C durchgeführt werden, wobei zusätzlich eine Mineralsäure als Katalysator eingesetzt werden kann.

REAKTIONSSCHEMA VIII

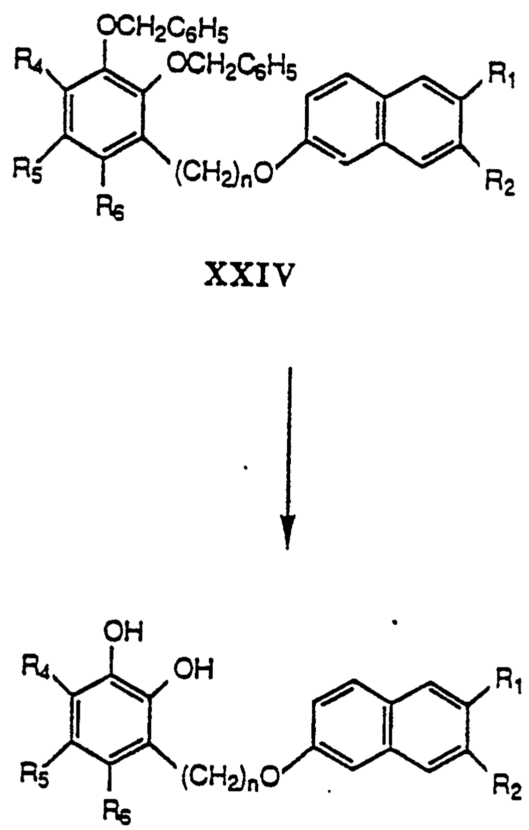

XXIV

Ia″

worin $R_1$, $R_2$, $R_4$, $R_5$, $R_6$ und n die obige Bedeutung haben.

Im Reaktionsschema VIII kann eine Verbindung der Formel XXIV in die entsprechende Verbindung der Formel Ia″ übergeführt werden, durch Schütteln in Wasserstoffatmosphäre bei Normaldruck und Raumtemperatur in Gegenwart eines Katalysators wie Palladium.

Es versteht sich, dass vorzugsweise, jedoch nicht notwendigerweise, jedes in den Reaktionsschemata I-VIII hergestellte Zwischenprodukt abgetrennt und nach bekannten Verfahren gereinigt werden kann, beispielsweise durch Ausfällen, Kristallisieren und Chromatographie, bevor es im nächsten Reaktionsschritt eingesetzt wird. Die Endprodukte der Formel I werden ebenfalls nach bekannten Verfahren abgetrennt. Die Erfindung betrifft ebenfalls Salze der Verbindungen der Formel I, falls R Wasserstoff ist, die durch Umsetzung der genannten Säure mit einer Base, die ein nicht toxisches, pharmazeutisch annehmbares Kation enthält, hergestellt werden können. Im allgemeinen kommt jede Base in Betracht, die ein Salz mit Carbonsäuren bildet und deren pharmakologische Eigenschaften keine abträglichen physiologischen Effekte verursachen.

Geeignete Basen sind beispielsweise Alkalimetall und Erdalkalimetallhydroxide und Carbonate, wie Kalziumhydroxid, Natriumhydroxid, Natriumcarbonat, Kaliumcarbonat, Ammoniak, primäre, sekundäre und

tertiäre Amine, wie Monoalkylamine, Dialkylamine, Trialkylamine, beispielsweise Methylamin, Diäthylamin, Triäthylamin, stickstoffhaltige heterocyclische Amine, beispielsweise Piperidin. Ein so hergestelltes Salz ist das funktionelle Aequivalent der entsprechenden Verbindung der Formel I, worin R Wasserstoff ist, und dem Fachmann ist klar, dass die Anzahl der Salze, die erfindungsgemäss hergestellt werden können, nur durch das Kriterium limitiert ist, dass die angewandte Base bei der Bildung der entsprechenden Salze nicht toxisch und physiologisch akzeptabel ist.

Es ist bekannt, dass der oxidative Stoffwechsel von Arachidonsäure auf dem $\Delta^5$-Lipoxygenase ($\Delta^5$-LO)-Weg zu Peptidoleukotrienen (($LTC_4$ und $LTD_4$) und Leukotrien $B_4$ ($LTB_4$) führt. $LTC_4$ und $LTD_4$ sind potente Bronchokonstriktoren der menschlichen Bronchien und tragen bei gewissen Spezies durch Erhöhung der Kapillarenpermeabilität zur Oedembildung bei. $LTB_4$ ist ein potenter chemotaktischer Faktor für Entzündungszellen. $LTB_4$ ist auch in Synovialflüssigkeit aus Patienten mit rheumatoider Arthritis und Gicht gefunden worden und kann ein Mediator von Entzündungen und Gelenkzerstörungen bei diesen Krankheiten sein. Infolgedessen können Hemmer des $\Delta^5$-LO-Wegs von therapeutischem Wert bei der Behandlung von Asthma und entzündlichen Erkrankungen sein.

Produkte des $\Delta^5$-LO-Weges ($LTB_4$, $LTC_4$, $LTD_4$) sind weiterhin in erhöhten Spiegeln bei Hautläsionen von Patienten mit Psoriasis und atopischer Dermatitis vorhanden und können Mediatoren dieser Hauterkrankungen sein. Die intrakutane Verabreichung von $LTB_4$ in die menschliche Haut führt zu einer Quaddelbildung und Rötung, gefolgt von Infiltration Neutrophiler in die Applikationsstelle. Der Einstrom Neutrophiler wird ebenfalls bei entzündlichen Reaktionen, die mit psoriatischen Läsionen verbunden sind, beobachtet. Die topische Anwendung von $LTB_4$ auf menschliche Haut verursacht Abszesse, ähnlich denen bei pustulärer Psoriasis.

Die Verbindungen der Formel I weisen beispielsweise eine Aktivität als $\Delta^5$-Lipoxygenasehemmer auf wie im nachfolgenden dargelegt wird. Die nützlichen pharmakologischen Eigenschaften der Verbindungen der Formel I können anhand der nachstehend ausgeführten Versuche gezeigt werden.

Die Verbindungen der Formel I sind von Wert als Mittel zur Behandlung entzündlicher Erkrankungen wie Arthritis, entzündlicher Darmerkrankungen wie Colitis und anderer Erkrankungen die nachstehend beschrieben sind, und als anti inflammatorische Mittel zur topischen Behandlung Leukotrienmediierter Hautentzündungen einschliesslich Psoriasis sowie bei bronchopulmonären Erkrankungen wie Asthma von Wert.

Das Syndrom der entzündlichen Darmerkrankung umfasst eine Vielzahl von Erkrankungen des Gastrointestinaltrakts wie Morbus Crohn des Colons und Ileums, ulzerative Colitis und Pseudomembran-Colitis. Gemeinsame Symptome dieser Erkrankungen sind Entzündungen des befallenen Bereiches der Mucosa des Gastrointestinaltraktes, Schleimhautgeschwüre, Oedeme, Infiltration der Mucosa mit Entzündungszellen und schwere Diarrhöe. Arachidonsäuremetaboliten des $\Delta^5$-LO--Weges werden als Mediatoren dieses Syndroms angesehen.

Nachstehend werden verschiedene Testmodelle zur Bestimmung der pharmakodynamischen Aktivität der erfindungsgemässen Verbindungen beschrieben.

## IN VITRO TEST FUER $\Delta^5$-LIPOXYGENASEHEMMER

Bei diesem Verfahren wird eine Verbindung auf ihren Effekt auf die $\Delta^5$-Lipoxygenase aus basophilen Leukämiezellen (RBL-1-Zellen) von Ratten geprüft. Die Aktivität des Enzyms wurde durch Messen der katalytischen Umwandlung von [1-$^{14}$C]Arachidonsäure in [1-$^{14}$C-5-Hydroperoxy-6,8,11,14-Eicosatetraensäure ([1-$^{14}$C]-5-HPETE) bestimmt, die zur Bildung des 5-Hydroxyderivats ([1-$^{14}$C]-5-HETE) führt. Der Test ist in Biochemical Pharmacology 32, 362 (1983) beschrieben.

Bei diesem Test zeigte die (S)-6-[6-(2,3-Dihydroxyphenyl)hexyloxy]α-methyl-2-naphthalinessigsäure einen $IC_{50}$-Wert von 0,012µM.

## IN VITRO TEST MIT PERITONEALEN MACROPHAGEN VON RATTEN

Dieser Test misst die Fähigkeit der Testverbindung, die Freisetzung von Arachidonsäure (AA) aus Phospholipidspeichern in der Plasmamembran freizusetzen und den anschliessenden Metabolismus der AA auf dem 5-LO und dem Cyclooxygenase (CO)-Weg zu den von den Zellen ausgeschiedenen Endprodukten Leukotrien $B_4$ ($LTB_4$, aus dem 5-LO-Weg) und Prostaglandin $E_2$ ($PGE_2$, aus dem CO-Weg).

Macrophagen wurden von Ratten durch Bauchfellwäsche mit Phosphat-gepufferter Kochsalzlösung minus $Ca^{+2}$ und $Mg^{+2}$ (PBS) erhalten. Die Zellen wurden dreimal mit PBS gewaschen und in Dulbecco's Modified Eagle medium (Gibco Laboratories) suspendiert, das L-Glutamin und D-Glucose enthielt und mit 10% fötalem Kälberserum ergänzt war. Die Zellen wurden mit einem Coulter ZBA-Zähler gezählt und dann in einer Konzentration von 4 x $10^6$ Zellen/ml resuspendiert. 3 ml der Zellsuspension wurden in Plastikkulturschalen (3 cm) gegeben und dann 90 Minuten bei 37°C festsetzen gelassen. Die Schalen wurden dreimal mit PBS gewaschen, um nichthaftende Zellen zu entfernen. $^{14}$C-AA ca. (54µCi/Mmol) wurden zugesetzt (1 µCi/Schale). Nach 90 Minuten wurde das nicht aufgenommene $^{14}$C-AA entfernt und die Zellschicht nochmals dreimal mit PBS gewaschen. Die Testverbindungen wurden in DMSO gelöst und mit phosphatgepufferter Hank's Lösung auf die entsprechende Konzentration gebracht. Die Zellen wurden mit der Testverbindung oder mit der zur Lösung der Testverbindungen verwendeten Lösung (Kontrolle) 30 Minuten bei 37°C inkubiert und dann 20 Minuten mit Kalziumionophor A 23187 ($5X10^{-7}$ M) stimuliert. Die extracelluläre Flüssigkeit wurde entfernt und die freigesetzte $^{14}$C Radioaktivität aus dem AA Metabolismus durch Flüssigscintillationsspektroskopie gemessen. Die Menge von $LTB_4$ und $PGE_2$ wurden in der extracellulären Flüssigkeit durch Radioimmunoassay mit spezifischen Antiseren gemessen. Die Wirksamkeit einer Testverbindung oder des Standards wurde als %-Hemmung des in Gegenwart von A 23187 produzierten maximalen Effektes berechnet und als 50%-ige Hemmkonzentration ($IC_{50}$) ausgedrückt.

Diese Versuchsanordnung misst die Hemmwirkung der Testverbindungen auf den 5-LO und den CO-Weg des AA-Stoffwechsels und diese Hemmung wird als $IC_{50}$-Wert für $LTB_4$ und $PGE_2$-Bildung ausgedrückt. Die mit einigen erfindungsgemässen Verbindungen erhaltenen Zahlwerte sind in Tabelle I dargestellt und zeigen, dass der Effekt vorwiegend auf dem 5-LO-Weg liegt.

ESSIGSAEURE-INDUZIERTE COLITIS BEI RATTEN IN VIVO

Dieser Test ist in Amer. J. Proc. Gastro. Col. Rec. Surg. 31: 11-18 (1980) und Gastroenterology 88: 55-63 (1985) und 86: 453-460 (1984) beschrieben worden. Die Essigsäureinduzierte Colitis ist gekennzeichnet durch ein Einwandern von Entzündungszellen in das Colon, wobei die Anzahl derartiger Zellen in der Mucosa durch die Aktivität der Myeloperoxidase gemessen wird, eines Marker-Enzyms für diese Zellen. Die wünschenswerte, positive Aktivität wird angezeigt durch eine Verminderung hoher Myeloperoxidasespiegel, die durch Essigsäure bewirkt wird. Männliche Ratten im Gewicht von 150-300 g wurden zweimal täglich während 2 Tagen entweder mit dem Vehikel (Wasser oder Dimethylsulfoxid) oder der geprüften Verbindung, in Wasser suspendiert oder in Dimethylsulfoxid suspendiert, durch orale Verabreichung vorbehandelt. Am dritten Tag wurden die Tiere in der gleichen Weise wie an den vorhergehenden beiden Tagen behandelt, mit Metofan anästhesiert, worauf 2 ml 2,5%-ige Essigsäure in das Darmlumen, unmittelbar gefolgt von 3 ml Luft und einer Spülung von 3 ml phosphatgepufferter Kochsalzlösung, injiziert wurden (die Essigsäure ist im Darmlumen hinreichend lange anwesend, um eine Entzündung zu verursachen, ohne jedoch schwere Necrosen oder irreversible Schäden hervorzurufen). Die Tiere erhielten dann eine zweite Dosis der Test verbindung in der gleichen Menge etwa 16 Stunden später. 24 Stunden nach der Behandlung mit Essigsäure wurden die Tiere getötet, die Darmschleimhaut wurde entfernt und bei pH 6 mit einem wässrigen Puffer homogenisiert, worauf die Myeloperoxidase im Homogenat mit o-Phenylendiamin als Chromagen nach dem ELISA-Assay (Zoological Soc., London, 1979, Seiten 29-30) gemessen wurde. Kontrolltiere wurden mit Vehikel und Kochsalzlösung anstelle von Essigsäure behandelt.

Die Messwerte für repräsentative erfindungsgemässe Verbindungen sind in Tabelle I angegeben.

Tabelle I

| Verbindung | Makrophagentest $IC_{50}$ ($\mu M$) | | | Essigsäure-Colitis | |
|---|---|---|---|---|---|
| | $^{14}C$ | $LTB_4$ | $PGE_2$ | Dosis mg/kg po | % Hemmung der Myeloperoxidaseansammlung |
| (S)-6-[6-(2,3-Dihydroxyphenyl)hexyloxy]-α-methyl-2-naphthalinessigsäure | 0.9 | 0.2 | 1 | 1 | 90 ± 11 |
| (S)-6-[6-(2,3-Dihydroxyphenyl)hexyloxy]-α-methyl-2-naphthalinessigsäureethylester | 3 | 0.6 | 4 | 10 | 23 ± 5 |
| (rac)-6-[6-(2,3-Dihydroxyphenyl)hexyloxy]-α-methyl-2-naphthalinessigsäure | | 0.6 | 3 | 10 | 88 ± 17 |
| 6-[6-(2,3-Dihydroxyphenyl)hexyloxy]-2-naphthalinessigsäure | 3 | 0.9 | 5 | 1 | 72 ± 8 |
| 6-[6-(2,3-Dihydroxyphenyl)hexyloxy]-2-naphthalincarbonsäure | 3 | 3 | 9 | 1 | 72 ± 8 |
| (S)-6-[6-(3,4-Dihydroxyphenyl)hexyloxy]-α-methyl-2-naphthalinessigsäure | 1 | 0.6 | 3 | 10 | 13 ± 2 |

Die Verbindungen der Formel I oder deren Salze oder Präparate, die eine therapeutisch wirksame Menge an Verbindungen der Formel I oder deren Salze enthalten, können nach an sich bekannten Methoden verabreicht werden, beispielsweise kann eine Verbindung der Formel I oder deren Salz entweder einzeln oder mit anderen pharmazeutischen Mittel, oral, parenteral, rektal oder durch Inhalieren, beispielsweise in Form eines Aerosols eines micronisierten Pulvers oder einer vernebelten Lösung verabreicht werden. Für die orale Verabreichung können Tabletten, Kapseln, beispielsweise im Gemisch mit Talk, Stärke, Milchzucker oder anderen inerten Inhaltsstoffen, d.h. pharmazeutisch anwendbaren Trägern, in Form von wässrigen Lösungen, Suspensionen, Elixieren oder wässrig alkoholischen Lösungen, beispielsweise im Gemisch von Zucker oder anderen Süsstoffen, Geschmacksstoffen, Farbstoffen, Verdickern und anderen konventionellen pharmazeutischen Excipientien oder als Kügelchen oral verabreicht werden. Für die parenterale Verabreichung kommen Lösungen oder Suspensionen in Betracht, beispielsweise wässrige Lösungen oder Lösungen in Erdnussöl oder Suspensionen mit Excipientien und Trägern, die für diese Verabreichungsart üblich sind. Für die rektale Verabreichung kommen Suppositorien mit inerten Trägern, wie Kakaobutter in Betracht. Für die topische Verabreichung können die Verbindungen der Formel I in Salben, Crèmes, Lotionen und Gele eingearbeitet werden. Im allgemeinen sind die Lösungen, Salben und Crèmes, die erfindungsgemäss angewandt werden können, auf der Basis von resorbierbaren und wasserlöslichen Stoffen oder Stoffen vom Emulsionstyp aufgebaut, wie Petrolatum, Lanolin und Polyäthylenglykolen.

Der hier verwendete Ausdruck "Verbindung der Formel I" schliesst, sofern zutreffend, Enantiomere und Racemate ein.

Lösungen können die Verbindung der Formel I in einem pharmazeutisch anwendbaren Lösungsmittel, wie Polyäthylenglykol enthalten.

Geeignete Lotionen sind echte Lösungen bis hin zu wässrig oder hydroalkoholischen Formulierungen mit fein verteilten Partikeln. Lotionen können Suspendierungs- oder Dispersionsmittel wie Cellulosederivate enthalten, beispielsweise Methylcellulose oder Aethylcellulose. Gele sind typischerweise halbfeste Präparate, die durch Gelieren einer Lösung oder einer Suspension einer Verbindung der Formel I in einem geeigneten wässrigen oder wasserfreien Vehikel unter Anwendung eines Geliermittels, wie Carboxypolymethylen erhalten werden und anschliessender Neutralisation auf die passende Konsistenz mit einem Alkalimetallhydroxid, beispielsweise Natriumhydroxid und einem Amin, beispielsweise Polyäthylencocoamin. Topische pharmazeutische Kompositionen mit einer Verbindung der Formel I können auch konventionelle Inhaltsstoffe wie Konservierungsstoffe, Stabilisatoren, Befeuchtungsmittel, Emulgatoren und Puffer in üblichen Mengen enthalten, die auf die jeweiligen Erfordernisse abgestellt sind und die vom Fachmann ohne weiteres bestimmt werden können.

Die zu verabreichende Dosis einer Verbindung der Formel I oder eines Salzes davon und die Häufigkeit der Verabreichung hängt von der Potenz und der Wirkungsdauer der jeweiligen Verbindung der Formel I und dessen Salz ab, wie auch von der gewählten Art der Verabreichung, der Schwere der Erkrankung und des Alters des zu behandelnden Subjekts. Orale Dosen einer Verbindung der Formel I oder eines Salzes davon können im Bereich von etwa 25 bis etwa 1000 mg pro Tag vorzugsweise betragen sie etwa 25 bis etwa 250 mg in einer oder in mehreren Dosen.

Da einige der Verbindungen der Formel I ein asymmetrisches C-Atom besitzen, werden sie gewöhnlich als Racemate erhalten. Die Spaltung solcher Racemate in die optisch aktiven Isomeren kann nach an sich bekannten Verfahren erfolgen. Manche racemische Gemische können einzeln kristallisiert und danach getrennt werden. Die chemische Trennung ist jedoch bevorzugt. Dabei werden Diastereomere aus einem Racemat einer Verbindung der Formel I mit einer optisch aktiven Verbindung gebildet. Die gebildeten Diastereomere werden durch selektive Kristallisation oder Chromatographie getrennt und in das entsprechende optische Isomer umgewandelt. Die Erfindung umfasst somit die Racemate der Verbindung der Formel I als auch deren optisch aktive Isomeren (Enantiomere).

In den nachfolgenden Beispielen sind alle Temperaturen in Celsiusgraden angegeben. Sofern nicht anders angegeben, wurden Extrakte über wasserfreiem Magnesiumsulfat getrocknet.

## Beispiel 1

Ein Gemisch von 4,9 g (S)-α-Methyl-6-[6-[2,3-bis(phenylmethoxy)phenyl]hexyloxy]-2-naphthalinessigsäure und 0,5 g 10% Palladium/Kohle in 100 ml Aethylacetat wurde 22 Stunden in einer Wasserstoffatmosphäre geschüttelt. Das Reaktionsgemisch wurde über Celit filtriert und das Filtrat unter vermindertem

Druck zu einem Oel eingeengt, das aus Aether/Hexan kristallisiert, 2,9 g (S)-[6-(2,3-Dihydroxyphenyl)-hexyloxy]-α-methyl-2-naphthalinessigsäure, Schmelzpunkt 136-139° lieferte.

Eine Lösung von 1,55M Butyllithium in Hexan (195 ml, 0,3 Mol) wurde im Verlauf von 30 Minuten tropfenweise zu einer gerührten Lösung von 1,2 Dimethoxybenzol (41,4 g, 0,3 Mol) in 700 ml wasserfreiem Tetrahydrofuran unter Argon bei Raumtemperatur gegeben. Das Reaktionsgemisch wurde unter Rühren 4 Stunden auf 40° erwärmt und dann auf -70° gekühlt. Danach wurde im Verlauf von 30 Minuten eine Lösung von 46 ml 1,6-Dibromhexan in 250 ml wasserfreiem Tetrahydrofuran tropfenweise zugesetzt. Das Kühlbad wurde entfernt und das Reaktionsgemisch eine Stunde gerührt und dann 4 Stunden auf 40° erwärmt. Das Lösungsmittel wurde zum grössten Teil entfernt, danach wurden 90 ml 3N Salzsäure zugesetzt und das Produkt mit Hexan extrahiert. Der Extrakt wurde mit Natriumbicarbonatlösung gewaschen, getrocknet und unter vermindertem Druck zu einem Oel eingeengt. Destillation lieferte 1-(6-Bromhexyl)-2,3-dimethoxybenzol als gelbes Oel (Siedepunkt 125-140/20 Pa).

Dieses Verfahren ist für die Herstellung von 1-(7-Bromheptyl)-2,3-dimethoxybenzol in J. Chem. Soc. Perkin I, 2331 (1980) beschrieben und wurde für die Herstellung aller Bromzwischenprodukte, in denen n = 3-10 ist, angewandt.

266 ml 1M Bortribromid in Methylenchlorid wurden tropfenweise im Verlauf von 1 Stunde zu einer auf -65° gekühlten Lösung von 40 g 1-(6-Bromhexyl)-2,3-dimethoxybenzol in 800 ml wasserfreiem Methylenchlorid unter Rühren in Argonatmosphäre gegeben. Das Kühlbad wurde dann entfernt und das Reaktionsgemisch 1,5 Stunden gerührt. Nach Kühlen im Eisbad wurden 100 ml Wasser und 50 ml 3N Salzsäure zugesetzt und das Gemisch 2 Stunden gerührt. Die organische Phase wurde abgetrennt, getrocknet und unter vermindertem Druck zu einem Oel eingeengt, das durch HPLC mit 5% Methanol-Chloroform gereinigt wurde. Man erhielt 34,7 g 1-(6-Bromhexyl)-2,3-dihydroxybenzol als Oel.

Ein Gemisch von 31,3 g 1-(6-Bromhexyl)-2,3-dihydroxybenzol, 41 ml Benzylbromid, 47,5 g Kaliumcarbonat und 4,4 ml Tris[2-(2-methoxyäthoxy)äthyl]amin (TDA-1) in 750 ml Toluol wurde 40 Stunden unter Rühren zum Rückfluss erhitzt. Das Reaktionsgemisch wurde mit halbgesättigter Kochsalzlösung gewaschen, getrocknet und unter vermindertem Druck zu einem Oel eingedampft, das durch HPLC mit 25% Toluol-Hexan gereinigt wurde. Man erhielt 34,6 g 1-(6-Bromhexyl)-2,3-bis (phenylmethoxy)benzol als Oel.

Zu einer Lösung von 11,3 g (S)-6-Methoxy-α-methyl-2-naphthalinessigsäure (Aldrich Chemical Company, Milwaukee, Wis., USA) in 300 ml Methylenchlorid wurden bei -70° 110 ml 1M Bortribromid im Verlauf von 10 Minuten gegeben. Das Reaktionsgemisch wurde auf 5° aufwärmen gelassen und 1 Stunde gerührt. Danach wurde 2 Stunden bei 24° gerührt und es wurden 100 ml Wasser und 25 ml 3N Salzsäure zugesetzt. Das Gemisch wurde 1,5 Stunden bei 24° gerührt, danach mit 200 ml Aether versetzt, die ätherische Phase abgetrennt, getrocknet und zu einem Feststoff konzentriert. Umkristallisation aus Aether-Methylenchlorid lieferte 8,7 g (S)-6-Hydroxy-α-methyl-2-naphthalinessigsäure, Smp. 170-180°.

Eine Lösung von 3,9 g (S)-6-Hydroxy-α-methyl-2-naphthalinessigsäure in 50 ml Methanol und 0,5 ml konzentrierter Schwefelsäure wurde 24 Stunden unter Rühren zum Rückfluss erhitzt. Das Methanol wurde unter vermindertem Druck entfernt und der Rückstand in Aethylacetat gelöst und die Lösung mit Natriumbicarbonatlösung gewaschen, getrocknet und unter vermindertem Druck zu einem Oel eingedampft. Reinigung durch Chromatographie an Silicagel mit 25% Aethylacetat-Hexan lieferte 3,7 g (S)-6-Hydroxy-α-methyl-2-naphthalinessigsäure-methylester. Smp. 82-85°, $[\alpha]_D$ +68,6° (in Chloroform).

Ein Gemisch von 3,25 g 1-(6-Bromhexyl)-2,3-bis(phenylmethoxy)benzol, 1,5 g (S)-α-Methyl-6-hydroxy-2-naphthalinessigsäure-methylester, 1,08 g Natriumjodid und 2,7 g Kaliumcarbonat in 60 ml Aceton und 6 ml Dimethylformamid wurde 48 Stunden unter Rühren zum Rückfluss erhitzt. Das Reaktionsgemisch wurde filtriert und das Filtrat unter vermindertem Druck konzentriert. Der Rückstand wurde durch HPLC mit 20% Aethylacetat-Hexan gereinigt und lieferte 3,24 g (S)-α-Methyl-6-[6-[2,3-bis(phenylmethoxy)phenyl] hexyloxy] -2-naphthalinessigsäure-methylester als Oel. Die NMR und Massenspektren entsprachen der Struktur.

Eine Lösung von 5,5 g (S)-α-Methyl-6-[6-[2,3-bis(phenylmethoxy)phenyl]hexyloxy] -2-naphthalinessigsäure-methylester in 135 ml Methanol und 45 ml 1N Natronlauge wurde 3 Stunden zum Rückfluss erhitzt. Das Lösungsmittel wurde unter vermindertem Druck entfernt und der Rückstand angesäuert und mit Aether extrahiert. Der getrocknete Extrakt wurde unter vermindertem Druck zu einem Oel konzentriert, das aus Aether-Hexan kristallisiert wurde und 4,9 g (S)-α-Methyl-6-[6-[2,3-bis(phenylmethoxy)-phenyl]hexyloxy]-2-naphthalinessigsäure, Smp. 66-70°, lieferte.

## Beispiel 2

Ein Gemisch von 1,2 g (S)-α-Methyl-6-[6-[2,3-bis(phenylmethoxy)phenyl]hexyloxy] -2-naphthalinessigsäureäthylester und 0,3 g 10% Palladium/Kohle in 50 ml Aethylacetat wurde 21 Stunden in einer Wasserstoffatmosphäre geschüttelt. Das Reaktionsgemisch wurde über Celit abfiltriert und das Filtrat unter vermindertem Druck zu einem Oel konzentriert. Kristallisation aus Aether-Hexan lieferte 0,59 g (S)-6-[6-(2,3-Dihydroxyphenyl)hexyloxy]-α-methyl-2-naphthalinessigsäure-äthylester als halbfesten Stoff.

Das Ausgangsmaterial wurde wie folgt erhalten:

Eine Lösung von 10,5 g (S)-6-Hydroxy-α-methyl-2-naphthalinessigsäure in 100 ml Aethanol und 1,5 ml konzentrierter Schwefelsäure wurde 48 Stunden zum Rückfluss erhitzt. Danach wurden 2,5 ml Schwefelsäure zugesetzt und es wurde weitere 24 Stunden zum Rückfluss erhitzt. Das Aethanol wurde unter vermindertem Druck entfernt, Aethylacetat wurde zugesetzt und die Lösung mit Natriumbicarbonatlösung gewaschen, getrocknet und unter vermindertem Druck zu einem Feststoff konzentriert. Reinigung durch Chromatographie an 300 g Silicagel mit 20% Aethylacetat-Hexan lieferte 8,0 g (S)-6-Hydroxy-α-methyl-2-naphthalinessigsäure-äthylester, Smp. 93-98°, $[\alpha]_D$ +39,5° (in Chloroform).

Ein Gemisch von 7,5 g 1-(6-Bromhexyl)-2,3-bis(phenylmethoxy)benzol, 3,7 g (S)-α-Methyl-6-hydroxy-2-naphthalinessigsäure-äthylester, 2,5 g Natriumjodid und 6,5 g Kaliumcarbonat in 200 ml Aceton und 200 ml Dimethylformamid wurde 43 Stunden unter Rühren zum Rückfluss erhitzt. Das Reaktionsgemisch wurde filtriert und das Filtrat unter vermindertem Druck konzentriert. Reinigung durch HPLC mit 10% Aethylacetat-Hexan lieferte 7,2 g (S)-α-Methyl-6-[6-[2,3-bis(phenylmethoxy)phenyl]hexyloxy] -2-naphthalinessigsäure-äthylester als Oel. Das Massenspektrum zeigte ein Molekülion bei m/e 616 ($C_{41}H_{44}O_5$), das NMR-Spektrum entsprach der Struktur.

## Beispiel 3

Ein Gemisch von 3,3 g rac-α-Methyl-6-[6-[2,3-bis(phenylmethyl)phenyl]hexyloxy] -2-naphthalinessigsäure und 0,8 g 10% Palladium/Kohle in 150 ml Aethylacetat wurde 19 Stunden unter Wasserstoffatmosphäre gerührt. Das Reaktionsgemisch wurde übe Celit filtriert und das Filtrat unter vermindertem Druck zu einem Feststoff konzentriert, der nach Umkristallisation aus Aether-Hexan 1,3 g rac-6-[6-(2,3-Dihydroxyphenyl)-hexyloxy]-α-methyl -2-naphthalinessigsäure, Smp. 134-143°, lieferte.

Das Ausgangsmaterial wurde wie folgt hergestellt:

Eine Suspension von 5,1 g rac-6-Methoxy-α-methyl-2-naphthalinessigsäure [J. Med. Chem. 13, 203 (1970)] in 50 ml Essigsäure und 25 ml 48%iger Bromwasserstoffsäure wurde mit gasförmigem Bromwasserstoff gesättigt und 3 Stunden unter Rühren zum Rückfluss erhitzt. Das Reaktionsgemisch wurde in Eiswasser gegossen und das Produkt abfiltriert, wobei 4,69 g rac-6-Hydroxy-α-methyl-2-naphthalinessigsäure, Smp. 165-170°, erhalten wurden.

Ein Gemisch von 4,67 g rac-6-Hydroxy-α-methyl-2-5-naphthalinessigsäure, 13,4 ml Methyljodid, 7,3 g Natriumbicarbonat in 50 ml Dimethylformamid wurde 24 Stunden unter Rühren auf 45° erwärmt. Das Lösungsmittel wurde an der Oelpumpe entfernt und der Rückstand mit Wasser behandelt und mit Aether extrahiert. Der getrocknete Extrakt wurde zu einem Oel konzentriert, das durch HPLC mit 15% Aethylacetat-Hexan gereinigt wurde. Man erhielt 5 g rac-6-Hydroxy-α-methyl-2-naphthalinessigsäure-methylester, Smp. 61-66°.

Ein Gemisch von 3,1 g 1-(6-Bromhexyl)-2,3-bis(phenylmethoxy)benzol, 1,5 g rac-6-Hydroxy-α-methyl-2-naphthalinessigsäure-methylester, 1,8 g Kaliumcarbonat und 0,98 g Natriumjodid in 35 ml Aceton und 10 ml Dimethylformamid wurde unter Rühren 43 Stunden zum Rückfluss erhitzt. Das Reaktionsgemisch wurde abfiltriert und das Filtrat unter vermindertem Druck konzentriert. Reinigung durch HPLC mit 10% Aethylacetat-Hexan lieferte 3,4 g rac-α-Methyl-6-[6-[2,3-bis(phenylmethoxy)phenyl]hexyloxy] -2-naphthalinessigsäure-methylester, Smp. 56-58°.

Eine Lösung von 3,35 g rac-α-Methyl-6-[6-[2,3-bis(phenylmethoxy)phenyl]hexyloxy] -2-naphthalinessigsäure-methylester in 100 ml Methanol und 3,7 ml 6N Natronlauge wurde 4 Stunden unter Rühren zum Rückfluss erhitzt. Das Lösungsmittel wurde unter vermindertem Druck entfernt, der Rückstand angesäuert und das Produkt filtriert, wobei rac-α-Methyl-6-[6-[2,3-bis(phenylmethoxy)phenyl]hexyloxy]-2-naphthalinessigsäure erhalten wurde. Ein Analysenpräparat wurde durch Umkristallisation aus Aether-Hexan erhalten.

## Beispiel 4

Ein Gemisch von 2,57 g 6-[6-[2,3-Bis(phenylmethoxy)phenyl]hexyloxy] -2-naphthalinessigsäure und 0,7 g 10% Palladium/Kohle in 125 ml Aethylacetat wurde 22 Stunden in einer Wasserstoffatmosphäre geschüttelt. Das Reaktionsgemisch wurde über Celit filtriert und das Filtrat unter vermindertem Druck zu einem Feststoff eingedampft, der aus Aether-Hexan umkristallisiert, 1,44 g 6-[6-(2,3-Dihydroxyphenyl)hexyloxy]-2-naphthalinessigsäure, Smp. 119-121°, lieferte.

Das Ausgangsmaterial wurde wie folgt erhalten:

Ein Gemisch von 4,55 g 6-Hydroxy-2-naphthalinessigsäure [Helv. Chim. Acta, 57, 790 (1974)], 7,6 g Natriumbicarbonat und 18 ml Aethyljodid in 50 ml Dimethylformamid wurde 18 Stunden unter Rühren auf 55° erwärmt. Das Lösungsmittel wurde unter vermindertem Druck entfernt, der Rückstand mit Wasser versetzt und das Rohprodukt filtriert und aus Aether-Hexan umkristallisiert. Man erhielt 4,49 g 6-Hydroxy-2-naphthalinessigsäure-äthylester vom Smp. 81-83°.

Ein Gemisch von 5,82 g 1-(6-Bromhexyl)-2,3-bis(phenylmethoxy)benzol, 2,7 g 6-Hydroxy-2-naphthalinessigsäure-äthylester, 3,3 g Kaliumcarbonat und 1,8 g Natriumjodid in 70 ml Aceton und 20 ml Dimethylformamid wurde 48 Stunden unter Rühren zum Rückfluss erhitzt. Das Reaktionsgemisch wurde abfiltriert, das Filtrat unter vermindertem Druck konzentriert und der Rückstand durch HPLC mit 10% Aethylactat-Hexan gereinigt, wobei 5,85 g 6-[6-[2,3-Bis(phenylmethoxy)phenyl]hexyloxy] -2-naphthalinessigsäure-äthylester, Smp. 53-55°, erhalten wurde.

Eine Lösung von 3 g 6-[6-[2,3-Bis(phenylmethoxy)phenyl]hexyloxy] -2-naphthalinessigsäure-äthylester in 75 ml Methanol und 3,3 ml 1N Natronlauge wurde 2 Stunden zum Rück fluss erhitzt. Das Lösungsmittel wurde unter vermindertem Druck entfernt und der Rückstand angesäuert und mit Aethylacetat extrahiert. Der trockene Extrakt wurde zu einem Feststoff konzentriert, der aus Methylenchlorid-Aether umkristallisiert wurde und 2,6 g 6-[6-[2,3-Bis(phenylmethoxy)phenyl]hexyloxy] -2-naphthalinessigsäure lieferte.

## Beispiel 5

Ein Gemisch von 2,6 g 6-[6-[2,3-Bis(phenylmethoxy)phenyl]hexyloxy] -2-naphthalincarbonsäure, 0,7 g 10% Palladium/Kohle in 125 ml Tetrahydrofuran wurde 17 Stunden in einer Wasserstoffatmosphäre geschüttelt. Das Reaktionsgemisch wurde über Celit filtriert und das Filtrat unter vermindertem Druck zu einem Feststoff konzentriert. Umkristallisation aus Aether-Hexan lieferte 1,34 g 6-[6-(2,3-Dihydroxyphenyl)-hexyloxy]-2-naphthalincarbonsäure, Smp. 167-171°.

Das Ausgangsmaterial wurde wie folgt hergestellt:

Ein Gemisch von 5,74 g 1-(6-Bromhexyl)-2,3-bis(phenylmethoxy)benzol, 2,5 g 6-Hydroxy-2-naphthalincarbonsäure-äthylester [J. Chem. Soc., 678 (1954)], 3,2 g Kaliumcarbonat und 1,75 g Natriumjodid in 70 ml Aceton und 20 ml Dimethylformamid wurde 48 Stunden unter Rühren zum Rückfluss erhitzt. Das Reaktionsgemisch wurde unter vermindertem Druck konzentriert, das Rohprodukt durch HPLC mit 10% Aethylacetat-Hexan gereinigt, wobei 6,26 g 6-[6-[2,3-Bis(phenylmethoxy)phenyl]hexyloxy] -2-naphthalincarbonsäure. Smp. 66-68°, erhalten wurden.

## Beispiel 6

Ein Gemisch von 1,2 g (S)-α-Methyl-6-[6-[3,4-bis(phenylmethoxy)phenyl]hexyloxy] -2-naphthalinessigsäure und 0,2 g 10% Palladium/Kohle in 35 ml Aethylacetat und 5 ml Tetrahydrofuran wurden 18 Stunden unter Wasserstoffatmosphäre gerührt. Das Reaktionsgemisch wurde über Celit filtriert und das Filtrat unter vermindertem Druck konzentriert. Der Rückstand wurde aus Aethylacetat-Hexan kristallisiert und lieferte 0,86 g (S)-6-[6-(3,4-Dihydroxyphenyl)hexyloxy]-α-methyl -2-naphthalinessigsäure, Smp. 103-106°.

Die Ausgangsverbindung wurde wie folgt erhalten:

Ein Gemisch von 1,0 ml 1,2-Dimethoxybenzol und 2 g 6-Bromhexansäure wurde kurz erwärmt, bis es homogen war und unter Rühren mit 1,7 ml Trifluoressigsäureanhydrid versetzt. Das Reaktionsgemisch wurde 17 Stunden bei Raumtemperatur gerührt und dann in Natriumbicarbonatlösung gegossen. Das Produkt wurde mit Aethylacetat extrahiert und der getrocknete Extrakt zu einem Oel konzentriert, das durch Chromatographie an 150 g Silicagel gereinigt wurde. Elution mit 25% Aethylacetat-Hexan lieferte 1,6 g 1-(6-Brom-1-oxohexyl)-3,4-dimethoxybenzol. Das NMR-Spektrum entsprach der Struktur.

Ein Gemisch von 32,9 g 1-(6-Brom-1-oxohexyl)-3,4-dimethoxybenzol, 3 g 10% Palldium/Kohle und 5 Tropfen konzentrierte Schwefelsäure in 200 ml Eisessig wurde 9 Stunden in einer Hydrierapparatur mit

einem Anfangsdruck von 4,7 bar geschüttelt. Das Reaktionsgemisch wurde über Celit abfiltriert und das Filrat unter vermindertem Druck konzentriert. Der Rückstand wurde durch HPLC mit 10% Aethylacetat-Hexan gereinigt und lieferte 28,7 g 1-(6-Bromhexyl)-3,4-dimethoxybenzol als Oel. Das NMR-Spektrum entsprach der Struktur und das LR-Massenspektrum zeigte ein Molekularion bei m/e 300 ($C_{14}H_{21}BrO_2$).

66 ml Bortribromid (1M in Methylenchlorid) wurde tropfenweise im Verlauf von 15 Minuten zu einer auf -70° gekühlten Lösung von 10 g 1-(6-Bromhexyl)-3,4-dimethoxybenzol in 200 ml Methylenchlorid unter Rühren in Argonatmosphäre gegeben. Das Reaktionsgemisch wurde 30 Minuten bei -70° und 3 Stunden bei 23° gerührt. Nach Kühlen im Eisbad wurden 40 ml Wasser und 20 ml 3N Salzsäure zugesetzt und das Gemisch wurde 1 Stunde bei 23° gerührt. Die organische Phase wurde abgetrennt, getrocknet und unter vermindertem Druck zu einem Oel konzentriert, das nach Umkristallisation aus Chloroform-Hexan 8,3 g 1-(6-Bromhexyl)-3,4-dihydroxybenzol, Smp. 60-62°, lieferte.

Ein Gemisch von 8,3 g 1-(6-Bromhexyl)-3,4-dihydroxybenzol, 18 ml Benzylbromid und 13 g Kaliumcarbonat in 160 ml Aceton und 50 ml Dimethylformamid wurde 24 Stunden unter Rühren zum Rückfluss erhitzt. Danach wurden 4 g Kaliumcarbonat zugesetzt und es wurde weitere 48 Stunden unter Rühren zum Rückfluss erhitzt. Das Reaktionsgemisch wurde gekühlt, filtriert und das Filtrat unter vermindertem Druck zu einem Oel eingeengt. Reinigung durch HPLC mit 1% Aethylacetat-Hexan lieferte 6,4 g 1-(6-Bromhexyl)-3,4-bis(phenylmethoxy)benzol. Das NMR-Spektrum entsprach der Struktur und das LR-Massenspektrum zeigte ein Molekularion bei m/e 452 ($C_{26}H_{29}BrO_2$).

Ein Gemisch von 2,2 g 1-(6-Bromhexyl)-3,4-bis(phenylmethoxy)benzol, 1,0 g (S)-α-Methyl-6-hydroxy-2-naphthalinessigsäure-äthylester, 0,68 g Natriumjodid und 2,4 g Kaliumcarbonat in 50 ml Aceton und 50 ml Dimethylformamid wurde 23 Stunden unter Rühren zum Rückfluss erhitzt. Das Reaktionsgemisch wurde filtriert und das Filtrat unter vermindertem Druck konzentriert. Der Rückstand wurde durch HPLC mit 10% Aethylacetat-Hexan gereinigt und lieferte 2,2 g (S)-α-Methyl-6-[6-[3,4-bis(phenylmethoxy)phenyl]hexyloxy]-2-naphthalinessigsäure-äthylester als Oel. Das NMR-Spektrum entsprach der Struktur.

Ein Gemisch von 2,2 g (S)-α-Methyl-6[6-[3,4-bis(phenylmethoxy)phenyl]hexyloxy] -2-naphthalinessigsäure-äthylester und 17 ml 1N Natronlauge in 50 ml Methanol und 17 ml Dioxan wurde 6 Stunden unter Rühren zum Rückfluss erhitzt. Danach wurden 100 ml Methanol und 1,2 ml 6N Natronlauge zugesetzt und das Gemisch wurde 7 Stunden unter Rühren zum Rückfluss erhitzt. Die Lösungsmittel wurden unter vermindertem Druck entfernt, der Rückstand angesäuert und das Produkt mit Aethylacetat extrahiert. Der getrocknete Extrakt wurde zu einem Oel konzentriert, das aus Aether-Hexan 1,25 g (S)-α-Methyl-6-[6-[3,4-bis(phenylmethoxy)phenyl]hexyloxy]-2-naphthalinessigsäure, Smp. 80-82°, lieferte.

## Beispiel A

| Tablettenformulierung (Feuchtgranulation) | | | |
|---|---|---|---|
| Inhaltsstoffe | mg/Tablette | | |
| | 100 mg | 500 mg | 1000 mg |
| 1. (S)-6-[6-(2,3-Dihydroxyphenyl)hexyloxy]-α-methyl-2-naphthalinessigsäure | 100 | 500 | 1000 |
| 2. Lactose | 132 | --- | --- |
| 3. Vorgelatinisierte Stärke | 16 | 30 | 50 |
| 4. Modifizierte Stärke | 30 | 40 | 50 |
| 5. Magnesiumstearat | 2 | 6 | 8 |
| Total | 280 | 576 | 1108 |

Herstellungsverfahren:

Die Inhaltsstoffe 1, 2, 3 und 4 werden mit Wasser granuliert. Das Granulat wird bei 50° getrocknet. Die granulierte Masse wird durch eine geeignete Mühle gegeben, danach wird der Inhaltsstoff 5 zugesetzt, es wird 3 Minuten gemischt und dann zu einer Tablette verpresst.

### Beispiel B

| Kapselformulierung | | | | |
|---|---|---|---|---|
| Inhaltsstoffe | mg/Kapsel | | | |
| 1. (S)-6-[-(6-2,3-Dihydroxyphenyl)hexyloxy]-α-methyl-2-naphthalinessigsäure | 25 | 50 | 100 | 500 |
| 2. wasserhaltige Lactose | 143 | 168 | 148 | --- |
| 3. Maisstärke | 20 | 20 | 40 | 70 |
| 4. Talk | 10 | 10 | 10 | 25 |
| 5. Magnesiumstearat | 2 | 2 | 2 | 5 |
| Total | 200 | 250 | 300 | 600 |

Herstellungsverfahren:

Die Inhaltsstoffe 1, 2 und 3 werden 30 Minuten gemischt, danach werden die Inhaltsstoffe 4 bis 5 zugegeben und es wird 3 Minuten gemischt und danach in Kapseln abgefüllt.

### Beispiel C

| Feuchtgranulat | | |
|---|---|---|
| Inhaltsstoffe | mg/Tablette | |
| 1. (S)-6-[6-2,3-Dihydroxyphenyl)hexyloxy]-α-methyl-2-naphthalinessigsäure | 25 | 50 |
| 2. Polyvinylpyrrolidon | 5 | 10 |
| 3. wasserfreie Lactose | 133 | 142 |
| 4. Avicel PH 102 | 25 | 30 |
| 5. Modifizierte Stärke | 10 | 15 |
| 6. Magnesiumstearat | 2 | 3 |
| Total | 200 | 250 |

Herstellungsverfahren:

Inhaltsstoff 2 wird in Wasser gelöst, die Inhaltsstoffe 1, 3, 4 und 5 werden gemischt und mit der Lösung von Inhaltsstoff 1 granuliert. Die Masse wird über Nacht bei 45° getrocknet, gesiebt, mit dem Inhaltsstoff 6 versetzt und gemischt und verpresst.

### Beispiel D

| Weichgelatinekapsel | | |
|---|---|---|
| Inhaltsstoffe | mg/Kapsel | |
| 1. (S)-6-[6-(2,3-Dihydroxyphenyl)hexyloxy]-α-methyl-2-naphthalinessigsäure | 50 | 250 |
| 2. PEG 400 | 325 | 550 |
| 3. MCM 90 | 100 | 150 |
| 4. Tween 80 | 25 | 50 |
| Total | 500 | 1000 |

Herstellungsverfahren:

Der Inhaltsstoff 1 wird in 2 gelöst, danach wird 3 und anschliessend 4 zugesetzt und es wird bis zur Lösung gemischt. Schliesslich wird in Weichgelatinekapseln abgefüllt.

Beispiel E

| 5%ige Crème | | |
|---|---|---|
| Inhaltsstoffe | g/kg | Variationsbreite |
| 1. (S)-6-[6-(2,3-Dihydroxyphenyl)hexyloxy]-α-methyl-2-naphthalinessigsäure | 51.50 | --- |
| 2. Glycerylmonostearat (Arlacel 165) | 100.00 | 80 - 120 |
| 3. Polysorbat 60 (Tween 60) | 20.00 | 15 - 25 |
| 4. Cetylalkohol | 50.00 | 40 - 60 |
| 5. Petrolatum | 70.00 | 50 - 90 |
| 6. Methylparaben | 1.50 | 1.25 - 1.75 |
| 7. Propylparaben | 0.50 | 0.4 - 0.6 |
| 8. Propylenglykol | 200.00 | 150 - 250 |
| 9. gereinigtes Wasser | 521.70 | 475 - 575 |
| Total | 1015.20 | |

Herstellungsverfahren:

Die Inhaltsstoffe 2, 3, 4 und 5 werden durch Erwärmen auf 80° geschmolzen. In einem getrennten Behälter wird 6 und 7 bei 80° in 9 gelöst. 1 wird bei 80° in 8 gelöst und zu 6 und 7 gegeben. Schliesslich wird zur erhaltenen Masse zur Schmelze von 2, 3, 4 und 5 gegeben und es wird gründlich gemischt. Danach wird langsam auf Raumtemperatur abkühlen gelassen.

Beispiel F

| Suppositorium | | | | |
|---|---|---|---|---|
| Inhaltsstoffe | g/Suppositorium | | | |
| 1. (S)-6-[6-(2,3-Dihydroxyphenyl)hexyloxyl]-α-methyl-2-naphthalinessigsäure | .025 | 0.10 | 0.50 | 1.00 |
| 2. Witepsol H15 | 1.975 | 1.90 | 1.50 | 1.00 |
| Total | 2.000 | 2.00 | 2.00 | 2.00 |

Herstellungsverfahren:

2 wird bei 50-55 °C geschmolzen. Danach wird 1 zugesetzt und es wird bis zur gleichmässigen Verteilung gemischt. Die so erhaltene Masse wird in Suppositorienformen gegossen und abkühlen gelassen.

Beispiel G

| Klistier | | | |
|---|---|---|---|
| Inhaltsstoffe | mg/5 ml | | |
| 1. (S)-6-[6-(2,3-Dihydroxyphenyl)hexyloxy]-α-methyl-2-naphthalinessigsäure | 25 | 100 | 500 |
| 2. Propyleneglykol | 1500 | 1500 | 1500 |
| 3. Methylparaben | 4 | 4 | 4 |
| 4. Propylparaben | 1 | 1 | 1 |
| 5. gereinigtes Wasser q.s. | 5 ml | 5 ml | 5 ml |

Herstellungsverfahren:

3 und 4 werden in 5 bei 80 °C gelöst. Danach wird auf 50° gekühlt und es wird 2 zugesetzt und auf Raumtemperatur abkühlen gelassen.

**Ansprüche**

1. Verbindungen der Formel

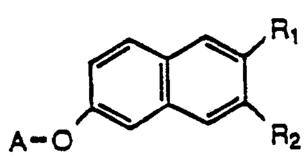

I

worin einer der Reste $R_1$ und $R_2$ $-(CHR_{10})_m-C(O)OR$ und der andere Wasserstoff ist, R Wasserstoff oder Niederalkyl, $R_{10}$ Wasserstoff oder Niederalkyl und m 0 oder 1 ist, A

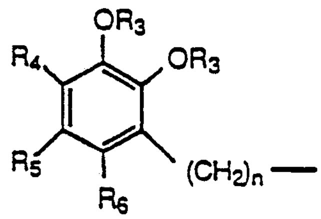

A'

ist, worin $R_3$ Wasserstoff oder Acyl, $R_4$ Wasserstoff, Halogen, Niederalkyl, Aryl oder Cycloalkyl, $R_5$ und $R_6$ unabhängig voneinander Wasserstoff oder Halogen und n eine ganze Zahl von 2-10 ist oder A ein Rest

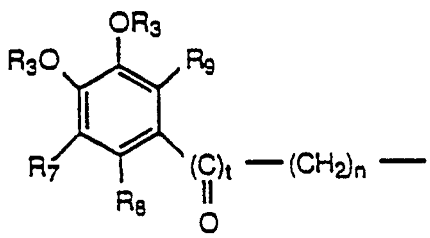

A"

ist, worin $R_3$ Wasserstoff oder Acyl, $R_7$ Wasserstoff oder Niederalkyl, $R_8$ und $R_9$ unabhängig voneinander Wasserstoff, Niederalkyl oder Halogen, t O oder 1 und n eine ganze Zahl von 2-10 ist
und falls $R_{10}$ Niederalkyl ist, Enantiomere und Racemate davon und, wenn R Wasserstoff ist, Salze davon mit pharmazeutisch anwendbaren Basen.

2. Verbindungen gemäss Anspruch 1, in denen A A′ ist, $R_1$ $-(CHR_{10})_m-C(O)OR$, $R_2$ Wasserstoff, $R_{10}$ Wasserstoff oder Methyl und $R_3$ Wasserstoff ist.

3. (S)-6-[6-(2,3-Dihydroxyphenyl)hexyloxy]-α-methyl-2-naphthalinessigsäure.

4. (S)-6-[6-(2,3-Dihydroxyphenyl)hexyloxy]-α-methyl-2-naphthalinessigsäureäthylester;
(Rac)-6-[6-(2,3-Dihydroxyphenyl)hexyloxy]-α-methyl-2-naphthalinessigsäure;
6-[6-(2,3-Dihydroxyphenyl)hexyloxy]-2-naphthalinessigsäure;
6-[6-(2,3-Dihydroxyphenyl)hexyloxy]-2-naphthalincarbonsäure und
(S)-6-[6-(3,4-Dihydroxyphenyl)hexyloxy]-α-ethyl-2-naphthalinessigsäure.

5. Die Verbindungen gemäss den Ansprüchen 1-4 als Heilmittel.

6. Die Verbindungen gemäss den Ansprüchen 1-4 zur Anwendung bei der Herstellung von Heilmitteln zur Behandlung entzündlicher Erkrankungen wie Arthritis, entzündlicher Hauterkrankungen wie Psoriasis und bronchopulmonären Erkrankungen wie Asthma.

7. Pharmazeutische Präparate enthaltend eine Verbindung gemäss den Ansprüchen 1-4 und einen Trägerstoff.

8. Verfahren zur Herstellung der Verbindungen der Formel I von Anspruch 1 und deren Salzen, dadurch gekennzeichnet, dass man
(a) eine Verbindung der Formel

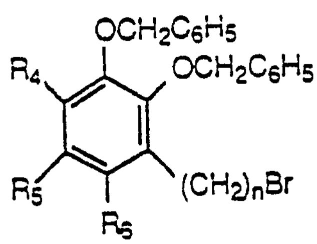

V

mit einer Verbindung der Formel

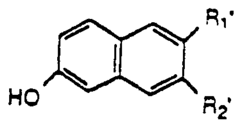

XXIII

zu einer Verbindung der Formel

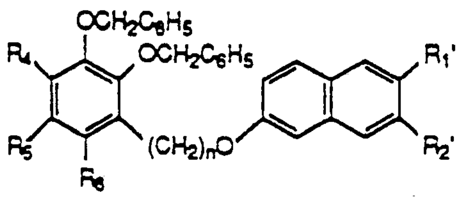

XXIVa

oder

(b) eine Verbindung der Formel

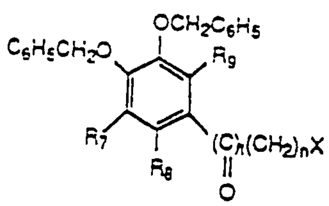

XXVI

mit einer Verbindung der Formel

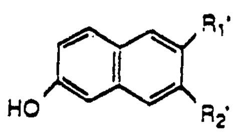

XXIII

zu einer Verbindung der Formel

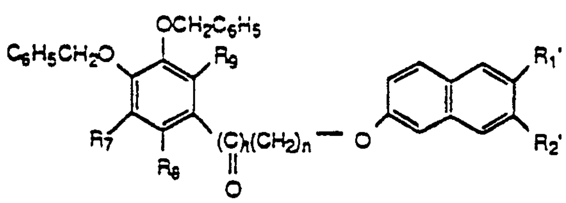

XXVII

umsetzt, wobei in den obigen Formeln einer der Reste $R_1'$ und $R_2'$ Wasserstoff und der andere $-(CHR_{10})_mCOOR'$ darstellt, worin $R'$ Niederalkyl ist, X Halogen ist und $R_4$, $R_5$ und $R_6$ m, n und t die obige Bedeutung haben; worauf man in beliebiger Reihenfolge die Benzyläthergruppen der Verbindungen der Formel XXIVa und XXVII und, gewünschtenfalls, eine Alkylestergruppe in den Gruppen $R_1'$ und $R_2'$ entfernt um eine Verbindung der Formel I zu erhalten worin $R_3$ Wasserstoff ist und $R_1$, $R_2$, $R_4$, $R_5$, $R_6$, m, n und t die obige Bedeutung haben und gewünschtenfalls phenolische Hydroxygruppen in der erhaltenen Verbindung der Formel I acyliert und weiterhin gewünschtenfalls eine erhaltene Carbonsäure in ein Salz überführt.